(19) Europäisches Patentamt
European Patent Office
Office européen des brevets

(11) EP 2 285 817 B1

(12) # FASCICULE DE BREVET EUROPEEN

(45) Date de publication et mention de la délivrance du brevet:
**01.08.2012 Bulletin 2012/31**

(21) Numéro de dépôt: **09745919.2**

(22) Date de dépôt: **05.05.2009**

(51) Int Cl.:
***C07H 5/04*** *(2006.01)* ***A61K 31/7008*** *(2006.01)*
***C07H 11/00*** *(2006.01)* ***A61P 9/00*** *(2006.01)*
***A61P 35/00*** *(2006.01)*

(86) Numéro de dépôt international:
**PCT/FR2009/000524**

(87) Numéro de publication internationale:
**WO 2009/138600 (19.11.2009 Gazette 2009/47)**

(54) **NOUVELLES UTILISATIONS DE DERIVES DE D-MANNOPYRANOSE ACTIVATEURS DE L'ANGIOGENESE**

NEUE VERWENDUNG VON ANGIOGENESE AKTIVIERENDEN D-MANNOPYRANOSEDERIVATEN

NOVEL USES OF D-MANNOPYRANOSE DERIVATIVES ACTIVATING ANGIOGENESIS

(84) Etats contractants désignés:
**AT BE BG CH CY CZ DE DK EE ES FI FR GB GR HR HU IE IS IT LI LT LU LV MC MK MT NL NO PL PT RO SE SI SK TR**

(30) Priorité: **07.05.2008 FR 0802538**

(43) Date de publication de la demande:
**23.02.2011 Bulletin 2011/08**

(73) Titulaire: **Centre National de la Recherche Scientifique**
**75016 Paris (FR)**

(72) Inventeurs:
- **MONTERO, Jean-Louis**
  **F-34270 Lauret (FR)**
- **MONTERO, Véronique**
  **F-34270 Lauret (FR)**
- **MOLES, Jean-Pierre**
  **F-34660 Cournonsec (FR)**
- **DE SANTA BARBARA, Pascal**
  **F-34690 Fabrègues (FR)**
- **JOVER, Bernard**
  **F-34080 Montpellier (FR)**

(74) Mandataire: **Noel, Chantal Odile et al**
**Cabinet Orès**
**36, rue de St Pétersbourg**
**75008 Paris (FR)**

(56) Documents cités:
**WO-A-99/58126 WO-A1-00/39139**

- **KHANJIN N A ET AL: "Synthesis of mannose-6-phosphate analogs: large-scale preparation of isosteric mannose-6-phosphonate via cyclic sulfate precursor" TETRAHEDRON LETTERS, ELSEVIER, AMSTERDAM, vol. 43, no. 22, 27 mai 2002 (2002-05-27), pages 4017-4020, XP004354624 ISSN: 0040-4039 cité dans la demande**
- **LIN ET AL: "Synthesis of sialyl Lewis x mimetics as selectin inhibitors by enzymic aldol condensation reactions" BIOORGANIC & MEDICINAL CHEMISTRY, ELSEVIER SCIENCE LTD, GB, vol. 7, no. 3, 1 janvier 1999 (1999-01-01) , pages 425-433, XP002232110 ISSN: 0968-0896**
- **A. PATEL, T.K. LINDHORST: "A modular approach for the synthesis of oligosaccharide mimetics" J. ORG. CHEM., vol. 66, 2001, pages 2674-2680, XP002512599**
- **BENITO J M ET AL: "Synthesis of 6,7-dideoxy-7-isothiocyanatoheptoses: stable fully unprotected monosacchardie isothiosyanates" CARBOHYDRATE RESEARCH, PERGAMON, GB, vol. 323, 1 janvier 2000 (2000-01-01), pages 218-225, XP002512601 ISSN: 0008-6215**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).



Printed by Jouve, 75001 PARIS (FR)

## Description

La présente Invention est relative à l'utilisation de certains dérivés de D-mannopyranoside pour le contrôle de l'angiogenèse. Ces composés présentent une activité pro-angiogénique et peuvent notamment être utilisés pour la préparation d'une composition pharmaceutique destinée au traitement des maladies cardiovasculaires ou au traitement de l'atrophie musculaire. L'invention est également relative, à certains dérivés D-mannopyranoside, à une composition pharmaceutique ou cosmétique comprenant ces dérivés et à l'utilisation d'une telle composition cosmétique pour prévenir et/ou traiter la chute des cheveux.

De nombreuses pathologies ont été décrites comme ayant une composante ou un stade lié au phénomène d'angiogénèse. On peut citer entre autres de très nombreux cancers, les rétinopathies liées au diabète, l'athérosclérose, l'arthrose, la polyarthrite rhumatoïde, le psoriasis, ainsi que les pathologies inflammatoires ou celles liées à une cicatrisation retardée.

L'angiogenèse est un mécanisme de néovascularisation prenant naissance à partir d'un réseau capillaire préexistant. Le bourgeonnement de petits vaisseaux, les capillaires, à partir de ceux préexistants, intervient pour le meilleur lors du développement embryonnaire et l'implantation du placenta, lorsqu'il s'agit de cicatriser une blessure, ou de pallier l'obstruction d'un vaisseau ; mais également pour le pire dans les cancers (croissance des tumeurs et développement des métastases), l'arthrite rhumatoïde, certaines maladies ophtalmologiques comme la rétinopathie diabétique ou la dégénérescence maculaire liée à l'âge... Pour l'ensemble de ces processus, le schéma général reste le même. L'activation des cellules endothéliales conduit à la dégradation de la membrane basale et de la matrice extracellulaire environnante. La migration orientée est suivie d'une phase proliférative. Les cellules se différencient ensuite en une structure de type capillaire pour former un réseau vasculaire nécessaire au développement des tissus. Au cours de ces dernières années, il est devenu clair que l'angiogenèse n'est pas contrôlée par un seul facteur, mais par une balance d'inducteurs et d'inhibiteurs produits par les cellules normales ou tumorales. Parmi ces facteurs, des polypeptides comme le facteur de croissance des fibroblastes-2 *("Fibroblast Growth Factor-2" :* FGF-2) et le "facteur de croissance de l'endothélium vasculaire *("Vascular Endothelial Growth Factor" :* VEGF) sont apparus comme étant des régulateurs clés de l'angiogenèse.

De nombreuses molécules ont été étudiées pour leur effet inhibiteur ou activateur de l'angiogénèse.

Par exemple, la demande internationale WO 99/58126 A décrit des dérivés de néomycine pour la préparation d'une composition pharmaceutique destinée à la prévention et/ou au traitement des pathologies dépendantes d'une activation de l'angiogénèse.

Par ailleurs, et en particulier, les molécules présentant une activité pro-angiogénique font l'objet de nombreuses recherches dans le domaine des pathologies cardiovasculaires. En effet, favoriser la formation de nouveaux vaisseaux collatéraux dans les tissus ischémiques en utilisant des molécules pro-angiogéniques (angiogenèse thérapeutique) représente un avenir très prometteur dans les maladies cardiovasculaires, notamment ischémiques. En effet, et malgré les progrès réalisés dans leur prévention et leurs traitements, les maladies cardiovasculaires ischémiques restent la première cause de morbidité et de mortalité dans les pays industrialisés. Les stratégies thérapeutiques actuelles qui visent à ralentir la progression de l'athérosclérose et à réduire la survenue des accidents thrombotiques ou les lésions d'ischémie ont certes gagné en efficacité mais la protection est encore insuffisante pour déclasser les maladies cardiovasculaires ischémiques. Il en est de même des stratégies qui consistent à réparer ou à remplacer : angioplasties, stents, pontages et autres greffes sauvent des vies mais la problématique d'ensemble demeure. Une autre approche thérapeutique consiste donc à favoriser la régénération de néovaisseaux de suppléance.

Les recherches effectuées jusqu'à ce jour dans ce domaine ont cependant conduit à des thérapies lourdes et complexes, telles que la thérapie génique qui pose en outre un réel problème de galénique, et/ou peu efficaces telles que l'utilisation de protéines recombinantes dont la biodisponibilité après administration ne donne pas toujours entièrement satisfaction (Lazarous DF, Shou M, Stiber JA, et al. Cardiovasc Res 1997 ; 36 : 78-85).

Il existe donc un réel besoin en molécules pro-angiogéniques faciles à synthétiser et à formuler afin de permettre leur utilisation notamment pour la préparation d'un médicament destiné à la prévention et/ou au traitement des maladies cardiovasculaires, en particulier ischémiques.

C'est donc afin de remédier à l'ensemble de ces inconvénients et de pourvoir à des composés ayant une activité pro-angiogénique pouvant notamment être utilisés pour la préparation d'un médicament pour le traitement des pathologies dans lesquelles une activation de l'angiogenèse est profitable que les Inventeurs ont mis au point ce qui fait l'objet de la présente Invention.

Les Inventeurs ont en effet découvert que certains dérivés sélectionnés de D-mannopyranoside tels qu'ils vont être décrits ci-après (composés de formule (I)) présentaient une activité pro-angiogénique, et qu'ils pouvaient par conséquent être notamment utilisés pour la préparation d'une composition pharmaceutique destinée au traitement des pathologies dans lesquelles une activation de l'angiogenèse est profitable telles que les pathologies cardiovasculaires et l'atrophie musculaire.

La présente Invention a donc pour objet l'utilisation, à titre de principe actif, d'au moins un composé de formule

(I) ci-après :

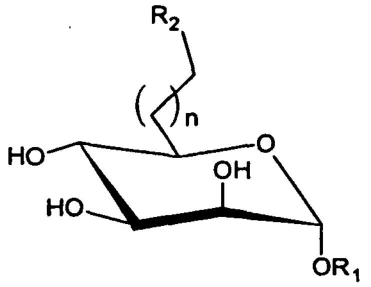

(I)

dans laquelle :

- $R_1$ représente un radical alkyle linéaire ou ramifié en $C_1$-$C_4$ ; un radical alkyle comportant un ou plusieurs groupements fonctionnels choisis parmi les groupements hydroxyle, amine, thiol, carboxyle, azide et nitrile ; un cycle hydrocarboné, saturé ou insaturé, en $C_3$-$C_6$ ; un cycle hydrocarboné, saturé ou insaturé, en $C_3$-$C_6$ comportant un ou plusieurs groupements fonctionnels choisis parmi les groupements hydroxyle, amine, alkyle en $C_1$-$C_4$, thiol, carboxyle, azide et nitrile ; un hétérocycle saturé ou insaturé comportant au moins un hétéroatome choisi parmi les atomes d'oxygène, d'azote et de souffre ;
- n est un nombre entier égal à 0 ou 1,
- $R_2$ est choisi parmi les groupements ($G_1$) à ($G_4$) suivants :

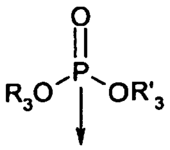

($G_1$)

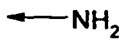

($G_2$)

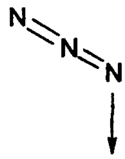

($G_3$)

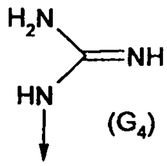

($G_4$)

dans lesquels :

* $R_3$ et $R'_3$, identiques ou différents, représentent un atome d'hydrogène ou de sodium ;
* la flèche représente le point d'attachement du groupement sur l'atome de carbone porteur de $R_2$,

pour la préparation d'une composition pharmaceutique destinée à la prévention et/ou au traitement des pathologies dépendantes d'une activation de l'angiogenèse.

Selon l'invention, parmi les radicaux alkyle en $C_1$-$C_4$ mentionnés pour $R_1$, le radical méthyle est particulièrement préféré.

Parmi les radicaux alkyle fonctionnalisés cités pour $R_1$, on peut en particulier mentionner les radicaux mono et dihydroxyalkyle en $C_1$-$C_4$, mono et diaminoalkyle en $C_1$-$C_4$, mono et dithioalkyle en $C_1$-$C_4$ et mono et dicarboxyalkyle en $C_1$-$C_4$.

Parmi les cycles hydrocarbonés cités pour $R_1$, on peut en particulier mentionner les cycles cyclopropane, cyclobutane, cyclopentane, cyclohexane, phényle, benzyle.

Parmi les hétérocycles cités pour $R_1$, on peut en particulier mentionner les cycles oxadiazole, triazole, oxazole, isoxazole, imidazole, thiadiazole, pyrrole, tetrazole, furane, thiophène, pyrazole, pyrazoline, pyrazolidine, thiazole, isothiazole, pyridine, pyrimidine, pipéridine, pyranne, pyrazine et pyridazine.

Dans les composés de formule (I) ci-dessus, lorsque n = 0, $R_2$ est de préférence un groupement $G_3$ et lorsque n = 1, $R_2$ est de préférence choisi parmi les groupements $G_1$, $G_2$ et $G_4$.

Selon une forme de réalisation préférée de l'invention, lorsque les composés de formule (I) sont choisis parmi ceux dans lesquels $R_2$ représente un groupement $G_1$ tel que défini ci-dessus, alors dans ledit groupement $G_1$, $R_3$ et $R'_3$ sont de préférence identiques et représentent un atome de sodium.

Parmi les composés de formule (I) ci-dessus, on peut en particulier citer :

- le 7-amino-6,7-didésoxy-D-manno-heptopyranoside de méthyle ;
- le 6-azido-6-déoxy-D-mannopyranoside de méthyle ;
- le 6-amino-6-deoxy-D-mannopyranoside de méthyle ;
- le 7-(disodium)phosphonato-6,7-didésoxy-D-manno-heptopyranoside de méthyle ;
- le 7-phosphonato-6,7-didésoxy-D-manno-heptopyranoside de méthyle.

Parmi ces composés, le 7-(disodium)phosphonato-6,7-didésoxy-D-manno-heptopyranoside de méthyle, le 6-azido-6-déoxy-D-mannopyranoside de méthyle et le 7-amino-6,7-didésoxy-D-manno-heptopyranoside de méthyle sont particulièrement préférés.

Certains des composés de formule (I) listés ci-dessus sont connus en tant que tels et ont déjà été proposés dans le domaine pharmaceutique, notamment pour améliorer la cicatrisation de la peau tout en diminuant la formation de cicatrices disgracieuses (Clavel, C. et al., Il Farmaco, 2005, 60, 721-725). Ils n'y ont cependant encore jamais été utilisés et aucune activité de ces composés sur la modulation de l'angiogénèse n'a encore été décrite. Leur effet concerne la prévention du désordre fibrotique.

Parmi les pathologies dans lesquelles une activation de l'angiogenèse est profitable, on peut tout particulièrement citer les pathologies cardiovasculaires, notamment ischémiques, ainsi que l'atrophie musculaire.

Lorsqu'elle est destinée à la prévention et/ou au traitement des pathologies cardiovasculaires, la composition pharmaceutique peut en outre renfermer, un ou plusieurs principes actifs additionnels utiles dans le traitement des maladies cardiovasculaires et parmi lesquels on peut notamment mentionner les anticoagulants fluidifiants tels que l'aspirine et l'héparine, les inhibiteurs du système rénine angiotensine, les béta bloquants, les inhibiteurs de l'hydroxyméthyl-glutaryl-coenzyme A (HMG CoA) synthase, etc...

Lorsqu'elle est destinée au traitement de l'atrophie musculaire, la composition pharmaceutique conforme à l'invention peut en outre renfermer un ou plusieurs principes actifs additionnels utiles dans le traitement de l'atrophie musculaire et parmi lesquels on peut notamment citer la somatotrophine (hormone de croissance humaine), l'érythropoïétine (EPO), les facteurs de croissance analogues à l'insuline ("Insuline-like Growth Factor" : IGF) tels que l'$IGF_1$ également connu sous la dénomination somatomédine C, les stéroïdes, etc...

Parmi les composés de formule (I) décrits ci-dessus, certains sont pour la première fois décrits pour une utilisation à titre de médicament.

Il s'agit des dérivés de D-mannopyranose de formule (I') suivante :

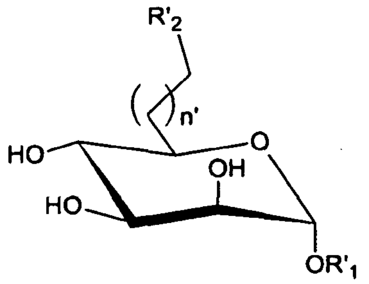

(I')

dans laquelle :

- $R'_1$ représente un radical alkyle fonctionnalisé choisi parmi les radicaux mono et dihydroxyalkyle en $C_1$-$C_4$, mono et diaminoalkyle en $C_1$-$C_4$, mono et dithioalkyle en $C_1$-$C_4$ et mono et dicarboxyalkyle en $C_1$-$C_4$ ; un cycle hydrocarboné choisi parmi les cycles cyclopropane, cyclobutane, cyclopentane, cyclohexane, phényle ; ou un hétérocycle choisi parmi les cycles oxadiazole, triazole, oxazole, isoxazole, imidazole, thiadiazole, pyrrole, tetrazole, furane, thiophène, pyrazole, pyrazoline, pyrazolidine, thiazole, isothiazole, pyridine, pyrimidine, pipéridine, pyranne, pyrazine et pyridazine ;
- n' est un nombre entier égal à 0 ou 1,
- $R'_2$ est choisi parmi les groupements ($G'_2$), ($G'_3$) et ($G'_4$) suivants :

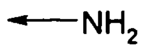

(G'$_2$)

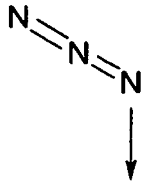

(G'$_3$)

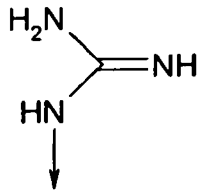

(G'$_4$)

dans lesquels :

* la flèche représente le point d'attachement du groupement sur l'atome de carbone porteur de R'$_2$.

Plus particulièrement, les composés de formule (I') ci-dessus sont appropriés pour une utilisation à titre de médicament pour la prévention et/ou le traitement des maladies dépendantes d'une activation de l'angiogénèse.
Les composés de formule (I') ci-dessus sont également particulièrement appropriés pour une utilisation à titre de médicament pour la prévention et/ou traitement des maladies cardiovasculaires et/ou de l'atrophie musculaire.
Parmi les radicaux alkyle fonctionnalisés cités pour R'$_1$, on peut en particulier mentionner les radicaux mono et dihydroxyalkyle en $C_1$-$C_4$, mono et diaminoalkyle en $C_1$-$C_4$, mono et dithioalkyle en $C_1$-$C_4$ et mono et dicarboxyalkyle en $C_1$-$C_4$.
Parmi les cycles hydrocarbonés cités pour R'$_1$, on peut mentionner les cycles cyclopropane, cyclobutane, cyclopentane, cyclohexane, phényle et benzyle.
Parmi les hétérocycles cités pour R'$_1$, on peut en particulier mentionner les cycles oxadiazole, triazole, oxazole, isoxazole, imidazole, thiadiazole, pyrrole, tetrazole, furane, thiophène, pyrazole, pyrazoline, pyrazolidine, thiazole, isothiazole, pyridine, pyrimidine, pipéridine, pyranne, pyrazine et pyridazine.
Dans les composés de formule (I') ci-dessus, lorsque n' = 0, R'$_2$ est de préférence un groupement G'$_3$ et lorsque n' = 1, R'$_2$ est de préférence un groupement G'$_2$ ou G'$_4$.
Selon une forme de réalisation préférée de l'invention, les composés de formule (I') ci-dessus sont choisis parmi ceux dans lesquels R'$_2$ représente un groupement G'$_3$.
Un autre objet de l'invention est une composition pharmaceutique caractérisée par le fait qu'elle comprend, à titre de principe actif, au moins un composé de formule (I') tel que défini ci-dessus et au moins un excipient pharmaceutiquement acceptable.
L'homme du métier choisira un ou plusieurs excipients pharmaceutiquement acceptables en fonction de la voie d'administration de la composition pharmaceutique. Bien entendu, l'homme de l'art veillera à cette occasion à ce que le ou les excipients utilisés soient compatibles avec les propriétés intrinsèques attachées à la composition conforme à la présente invention.
En outre, la forme du médicament ou de la composition pharmaceutique (par exemple, une solution, une suspension, une émulsion, des comprimés, des gélules, des suppositoires, etc...) dépendra de la voie d'administration choisie.
Ainsi, au sens de la présente Invention, le médicament ou la composition pharmaceutique peut être administré par n'importe quelle voie appropriée, par exemple par la voie orale, locale, systémique, intraveineuse, intramusculaire ou mucosale, ou bien en utilisant un patch.
On peut notamment citer, à titre d'exemples non limitatifs d'excipients appropriés pour une administration par voie orale, le talc, le lactose, l'amidon et ses dérivés, la cellulose et ses dérivés, les polyéthylèneglycols, les polymères d'acide acrylique, la gélatine, le stéarate de magnésium, des matières grasses animales, végétales ou synthétiques, les dérivés de la paraffine, les glycols, les stabilisants, les conservateurs, les anti-oxydants, les agents mouillants, les anti-agglomérants, les dispersants, les émulsionnants, les agents modifiants du goût, les agents de pénétrations, de solubilisation, etc....
Les techniques de formulation et d'administration des médicaments et compositions pharmaceutiques sont bien connues dans la technique ici considérée, l'homme du métier pouvant notamment se référer à l'ouvrage Remington's Pharmaceutical Sciences, dernière édition.
Les composés de formule (I) conformes à l'invention peuvent également être utilisés pour la préparation d'une composition cosmétique ou d'un complément alimentaire destiné à prévenir et/ou freiner la chute des cheveux, ce qui constitue un objet supplémentaire de la présente invention.
La présente invention a donc également pour objet une composition cosmétique caractérisée par le fait qu'elle comprend, dans un milieu cosmétiquement acceptable, au moins un composé de formule (I) telle que définie précédemment.

Cette composition se présente de préférence sous forme liquide, par exemple sous la forme d'une lotion ou d'un shampooing et peut être appliquée de façon topique directement sur le cuir chevelu, avec ou sans rinçage.

L'invention a pour objet un complément alimentaire caractérisé par le fait qu'il comprend au moins un composé de formule (I) telle que définie précédemment.

Ce complément alimentaire peut notamment se présenter sous la forme de gélules ou de comprimés et est destiné à être ingéré par voie orale.

Les composés de formule (I) (incluant les composés de formule (I')) peuvent être aisément préparés, à partir d'un D-mannopyranoside de formule (II) définie ci-après, par déplacement nucléophile du précurseur sulfate cyclique de formule (IV) correspondant, par analogie à la méthode décrite par exemple par Van der Klein P.A.M. et al., Carbohydr. Res., 1992, 224, 193-200 suivi de la déprotection des radicaux hydroxyle portés par le motif saccharidique, selon le schéma réactionnel A suivant :

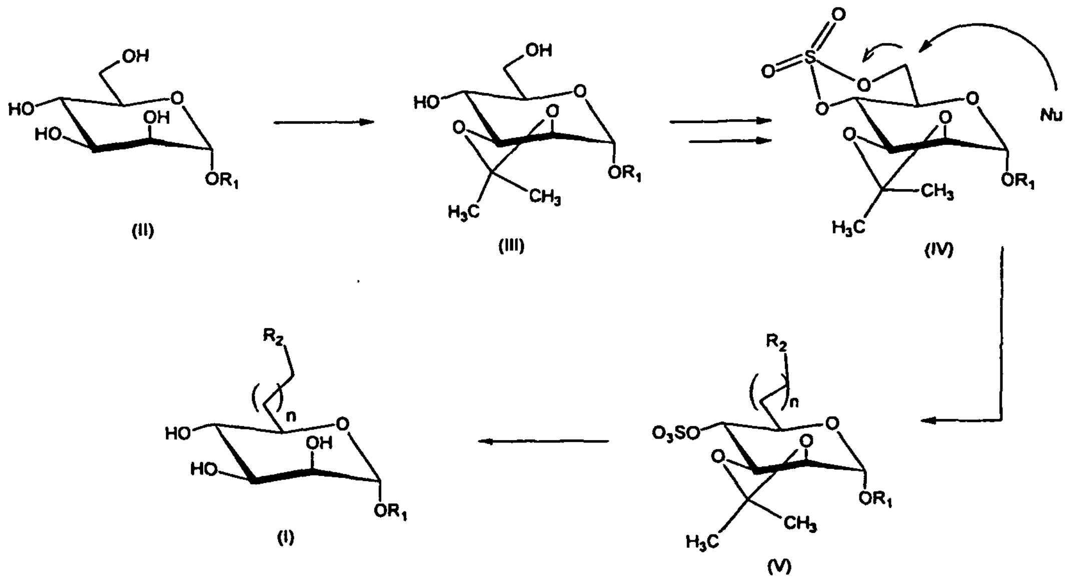

**SCHÉMA A**

dans lequel $R_1$, $R_2$ et n ont la même signification que celle indiquée ci-dessus pour les composés de formule (I) et Nu représente un groupement nucléophile correspondant au groupement $R_2$ que l'on souhaite introduire.

Cette méthode correspond à une adaptation de la méthode décrite dans l'article de Khanjin N.A. et a/., Tetrahedr. Lett., 2002, 43, 4017-4020.

Le sulfate cyclique de formule (IV) préparé selon ce procédé peut être stocké plusieurs mois à température ambiante sous la forme d'une poudre blanche sans observer de décomposition. Les intermédiaires purs des sels de monosulfate peuvent être facilement séparés des groupements nucléophiles n'ayant pas réagi et des autres impuretés, par partition entre l'eau et un solvant tel que le dichlorométhane avant l'étape de déprotection. Le clivage simultané et quantitatif des groupes monosulfate cyclique et isopropylidène des composés de formule (V) peut être réalisé sur une résine échangeuse d'ions telle qu'une résine Amberlyst-15 (H+) qui permet la déprotection du groupement monosulfate cyclique en 10 à 30 minutes et celle du groupement isopropylidène en 3 à 5 heures à température ambiante dans un mélange méthanol/tetrahydrofuranne. Tous les composés de formule (I) préparés selon ce procédé peuvent être obtenus avec un rendement compris entre 60 et 95 %.

Outre les dispositions qui précèdent, l'Invention comprend encore d'autres dispositions qui ressortiront de la description qui va suivre, qui se réfère à des exemples de préparation des composés de formule (I') conformes à l'invention, ainsi qu'à un exemple de mise en évidence de l'activité pro-angiogénique des composés de formule (I) par rapport à d'autres dérivés de D-mannopyranose ne répondant pas à la formule (I) et ne faisant donc pas partie de l'invention, ainsi qu'à la figure 1 annexée qui représente des photos de la vascularisation d'embryons de poulets après mise en culture en présence de 6 mg/ml de différents composés de formule (I) conformes à l'invention comparativement à quatre dérivés de D-mannopyranoside (DM) ayant une activité inhibitrice de l'angiogenèse et ne faisant donc pas partie de l'invention (**DM1** : le (disodium)-6-phosphate-D-manno-pyranoside de méthyle ; **DM2** : le 6,7-didéoxy-7-sulfonato-D-manno-heptopyranoside de méthyle ; **DM3** : le 6-deoxy-6-malonate-D-mannopyranoside de méthyle et **DM4** : l'acide (6,7-didésoxy-D-manno-heptopyranoside de méthyle) uronique)).

Il doit être entendu toutefois que ces exemples ne sont donnés qu'à titre purement illustratif de l'Invention dont

ils ne constituent en aucune manière une quelconque limitation.

**EXEMPLE 1 : PRÉPARATION DU 7-AMINO-6,7-DIDESOXY-α-D-*MANNO*-HEPTOPYRANOSIDE DE MÉTHYLE (Composé I-1)**

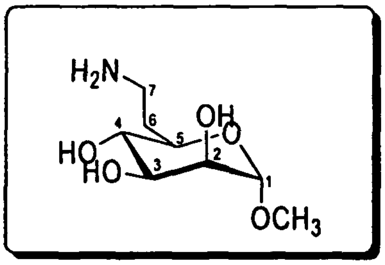

**(I-1)**

**1) Première étape : Préparation du 2,3-*O*-Isopropylidène-4,6-*O*-(sulfate cyclique)-α-D-mannopyranoside de méthyle (composé 3)**

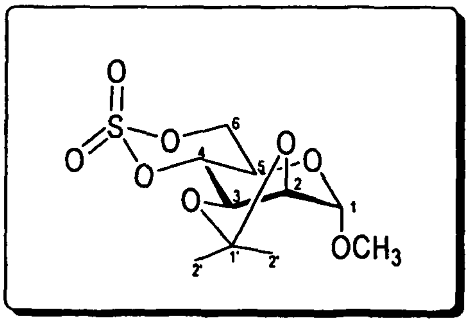

**(3)**

Le composé (3) a été obtenu en 2 sous-étapes, sans purification intermédiaire, via le sulfite correspondant (2).

**1-a) Préparation du sulfite correspondant (2)**

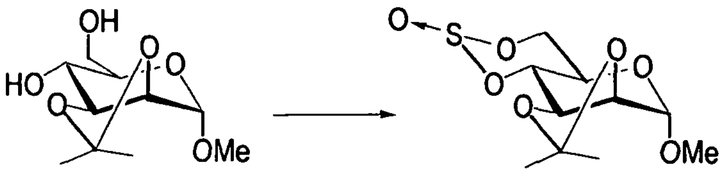

**(1)** **(2)**

3,79 g (16,18 mmol - 1 éq.) de 2,3-*O*-isopropylidène-α-D-mannopyranoside de méthyle **(1)** et 6,75 mL (48,54 mmol - 3 éq.) de triéthylamine ont été dissous dans 75 mL de dichlorométhane ($CH_2Cl_2$). Le mélange a été refroidi à 0°C et 1,3 mL (17,80 mmol - 1,1 éq.) de chlorure de thionyle ($SOCl_2$) ont été ajoutés lentement. Le précipité blanc de chlorure de triéthylammonium s'est formé instantanément, et le mélange réactionnel est devenu progressivement jaune, puis marron, en 5 à 10 minutes. Une chromatographie sur couche mince (CCM) a alors été réalisée en utilisant comme phase mobile un mélange d'éther de pétrole (EP) et d'acétate d'éthyle (AcOEt) (8/2 v/v). Les résultats de cette CCM ont indiqué alors qu'il ne restait plus de produit de départ (Rf=0) et que le sulfite désiré avait été obtenu sous forme de 2 diastéréoisomères (Rf=0,45 et 0,60). Le mélange réactionnel a alors été filtré, et la phase organique a été lavée avec de l'eau distillée, une solution d'acide chlorhydrique (HCl) 1N, et de l'eau distillée à nouveau. Elle a été séchée sur

sulfate de sodium ($Na_2SO_4$), filtrée et concentrée pour donner un solide légèrement marron qui a été directement remis en réaction.

**1-b) Oxydation du sulfite (2) en sulfate (3)**

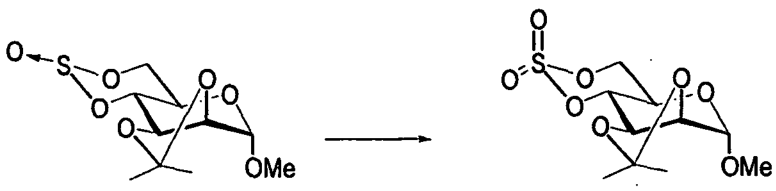

(2) (3)

Le sulfite brut (2) obtenu ci-dessus à la sous étape 1-a) (16,18 mmol - 1 éq., théoriquement) a été dissous dans 60 mL d'une solution composée d'un mélange de $CH_2Cl_2$ et d'acétonitrile ($CH_3CN$) (1/1 v/v) avant d'ajouter successivement 3,8 g (17,80 mmol - 1,1 éq.) de métaperiodate de sodium, 20 mL d'eau et 14 mg (0,06 mmol - 0,004 éq.) de chlorure de ruthénium. La réaction a été exothermique, et la formation du précipité d'iodate de sodium ($NaIO_3$) a été observée très rapidement. Après 1 heure de réaction, il ne restait plus de sulfite et seul le sulfate (3) a été observé sur CCM. Le mélange réactionnel a alors été filtré et dilué avec 100 mL de $CH_2Cl_2$. L'eau résiduelle de la réaction a été supprimée et la phase organique a été lavée 2 fois avec une solution de bicarbonate de sodium ($NaHCO_3$) à 5%, puis avec de l'eau distillée. Elle a ensuite été séchée sur $Na_2SO_4$, filtrée et concentrée pour donner un solide légèrement marron.

Ce solide a été dissous dans un minimum de $CH_2Cl_2$ en présence de charbon actif, et filtré sur silice. La silice a été rincée avec 300 mL de $CH_2Cl_2$. Les impuretés marron, contenant les sels de ruthénium, sont restés à la surface. Le solide blanc obtenu a ensuite été engagé dans l'étape 2) sans aucune autre purification.

**Rendement** : 84 % sur 2 étapes.

**Rf** : 0,48 (EP/AcOEt 7/3 v/v).

**SM : (ESI$^+$/MeOH)** m/z : 297 $[M+H]^+$, 319 $[M+Na]^+$.

**RMN $^1$H (400,13 MHz, Acétone-d$_6$) δ ppm :** 1,38 et 1,53 (2s, 6H, $H_{2'}$); 3,46 (s, 3H, $OCH_3$) ; 4,17 (td, 1H, $J_{5-4}$ = $J_{5-6b}$ = 10,6 Hz, $J_{5-6a}$ = 5,5 Hz, $H_5$) ; 4,32 (dd, 1H, $J_{2-3}$ = 5,6 Hz, $J_{2-1}$ = 0,4 Hz, $H_2$) ; 4,42 (dd, 1H, $J_{3-2}$ = 5,6 Hz, $J_{3-4}$ = 7,7 Hz, $H_3$) ; 4,59 (dd, 1H, $J_{4-3}$ = 7,8 Hz, $J_{4-5}$ = 10,4 Hz, $H_4$) ; 4,64 (t, 1H, $J_{6b-5}$ = 10,7 Hz, $J_{6b-6a}$ = -10,7 Hz, $H_{6b}$) ; 4,87 (dd, 1H, $J_{6a-5}$ = 5,5 Hz, $J_{6a-6b}$ = -10,5 Hz, $H_{6a}$) ; 5,01 (d, 1H, $J_{1-2}$ = 0,5 Hz, $H_1$).

**RMN $^{13}$C (100,62 MHz, CDCl$_3$) δ ppm :** 26,4 et 28,3 (2C, $C_{2'}$) ; 56,1 (1C, $OCH_3$) ; 58,9 (1C, $C_5$) ; 72,3 (1C, $C_6$) ; 73,6 (1C, $C_3$) ; 76,3 (1C, $C_2$) ; 84,6 (1C, $C_4$) ; 99,4 (1C, $C_1$) ; 111,0 (1C, $C_{1'}$).

**2) Deuxième étape : Préparation 6-cyano-6-déoxy-4-*O*-sodiumsulfate-2,3-*O*-isopropylidène-α-D-manno-pyranoside de méthyle (composé 4).**

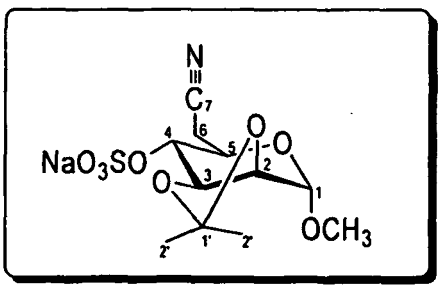

(4)

1 g (3,38 mmol - 1 éq.) de 2,3-*O*-Isopropylidène-4,6-*O*-(sulfate cyclique)-α-D-mannopyranoside de méthyle (composé 3) tel qu'obtenu ci-dessus à l'issue de l'étape 1) a été est dissous dans 3 mL de diméthylformamide (DMF), avant d'ajouter 331 mg (6,75 mmol - 2 éq.) de cyanure de sodium. Le mélange a été laissé sous agitation magnétique à température ambiante pendant 20 heures. Le milieu réactionnel a alors été dilué avec 20 mL de $NaHCO_3$ à 1% (pour éviter un éventuel dégagement de cyanure d'hydrogène (HCN), et lavé avec 10 mL de $CH_2Cl_2$. Le produit a encore été extrait de la phase organique avec 2 x 10 mL d'eau distillée. Les phases aqueuses rassemblées ont été lyophilisées pour donner un solide un peu jaune, qui était assez pur pour être remis en réaction directement. Cependant, ce produit peut également être purifié par chromatographie sur gel de silice avec un gradient d'élution ($CH_2Cl_2$ jusqu'à $CH_2Cl_2$/MeOH 91/9 v/v) pour donner une mousse très légèrement jaune.

**Rendement** : Quantitatif.

**Rf** : 0,49 ($CH_2Cl_2$/MeOH 85/15 v/v).

Le produit s'est révélé de couleur bordeaux à l'anisaldéhyde.

**SM ($ESI^+$/MeOH)** m/z : 384 $[M+Na]^+$.

**SM ($ESI^-$/MeOH)** m/z : 322 $[M-Na]^-$.

**RMN $^1H$ (400,13 MHz, Acétone-$d_6$) δ ppm :** 1,24 et 1,41 (2s, 6H, $H_{2'}$) ; 2,76 (dd, 1H, $J_{6a-5}$ = 9,3 Hz, $J_{6a-6b}$ = - 17,3 Hz, $H_{6a}$) ; 3,18 (dd, 1H, $J_{6b-5}$ = 2,8 Hz, $J_{6b-6a}$ = - 17,3 Hz, $H_{6b}$) ; 3,46 (s, 3H, $OCH_3$) ; 3,86 (td, 1H, $J_{5-6a}$ = $J_{5-4}$ = 9,6 Hz, $J_{5-6b}$ = 2,8 Hz, $H_5$) ; 4,15 (d, 1H, $J_{2-3}$ = 7,4 Hz, $H_2$) ; 4,21 (dd, 1H, $J_{4-5}$ = 9,9 Hz, $J_{4-3}$ = 7,0 Hz, $H_4$) ; 4,44 ($dd_{mal\ résolu}$, 1H, $H_4$); 4,93 (s, 1H, $H_1$).

**RMN $^{13}C$ (100,62 MHz, Acétone-$d_6$) δ ppm :** 20,6 (1C, $C_6$) ; 25,5 et 27,1 (2C, $C_{2'}$) ; 54,5 (1C, $OCH_3$) ; 64,9 (1C, $C_5$) ; 75,6 (1C, $C_2$) ; 76,3 (1C, $C_4$) ; 76,9 (1C, $C_3$) ; 98,1 (1C, $C_1$) ; 109,8 (1C, $C_{1'}$) ; 118,1 (1C, $C_7$).

<u>3) Troisième étape : Préparation du 6-cyano-6-déoxy-α-D-mannopyranoside de méthyle (composé 5)</u>

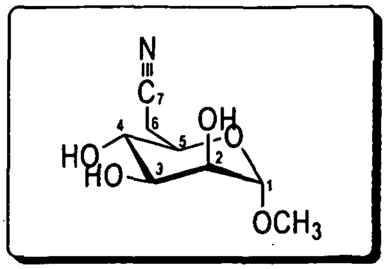

**(5)**

873 mg (2,53 mmol - 1 éq.) de 6-déoxy-6-cyano-4-sodiumsulfate-2,3-O-isopropylidène-α-D-manno-heptopyranoside de méthyle (4) obtenu ci-dessus à l'étape précédente ont été dissous dans 20 mL d'une solution constituée d'un mélange de méthanol (MeOH) et de THF (1/1 ; v/v), puis 1 g de résine Amberlyst-15 $H^+$ ont été ajoutés. Après 1 heure et 15 min de réaction, les résines ont été filtrées et le milieu réactionnel a été neutralisé avec une solution de $NaHCO_3$ à 5% jusqu'à pH = 8. Les solvants organiques ont été éliminés à l'évaporateur rotatif et l'eau restante a été lyophilisée. Le mélange a été repris au MeOH, et le $NaHCO_3$ insoluble a été filtré. Le produit a ensuite été purifié par chromatographie sur gel de silice avec un gradient d'élution ($CH_2Cl_2$ jusqu'à $CH_2Cl_2$/MeOH 92/8 v/v) pour donner une mousse blanche.

**Rendement** : 72 %.

**Rf** : 0,56 ($CH_2Cl_2$/MeOH 85/15 v/v).

**SM : ($ESI^+$/MeOH)** m/z : 226 $[M+Na]^+$, 242 $[M+K]^+$, 429 $[2M+Na]^+$.

**RMN $^1H$ (400,13 MHz, $D_2O$) δ ppm :** 2,86 (dd, 1H, $J_{6a-5}$ = 7,4 Hz, $J_{6a-6b}$ = - 17,3 Hz, $H_{6a}$) ; 3,04 (dd, 1H, $J_{6b-5}$ = 3,6 Hz, $J_{6b-6a}$ = - 17,3 Hz, $H_{6b}$) ; 3,44 (s, 3H, $OCH_3$) ; 3,60 (t, 1H, $J_{4-5}$ = $J_{4-3}$ = 9,7 Hz, $H_4$); 3,76 (dd, 1H, $J_{3-4}$ = 9,6 Hz, $J_{3-2}$ = 3,4 Hz, $H_3$) ; 3,84 (ddd, 1H, $J_{5-6a}$ = 7,1 Hz, $J_{5-6b}$ = 3,2 Hz, $J_{5-4}$ = 10,1 Hz, $H_5$) ; 3,96 (dd, 1H, $J_{2-3}$ = 3,4 Hz, $J_{2-1}$ = 1,7 Hz, $H_2$) ; 4,78 (d, 1H, $J_{1-2}$ = 1,5 Hz, $H_1$).

**RMN $^{13}C$ (100,62 MHz, $D_2O$) δ ppm :** 51,4 (1C, $C_6$) ; 55,2 (1C, $OCH_3$) ; 67,8 (1C, $C_5$) ; 70,2 (1C, $C_2$) ;

70,7 (1C, $C_3$) ; 71,6 (1C, $C_4$) ; 101,4 (1C, $C_1$).

**4) Quatrième étape : Préparation du 7-amino-6,7-didesoxy-α-D-*manno*-heptopyranoside de méthyle (composé I-1)**

450 mg (2,21 mmol - 1 éq.) de 6-déoxy-6-cyano-α-D-manno-heptopyranoside de méthyle (5) tel qu'obtenu ci-dessus à l'issue de l'étape 3) ont été dissous dans 10 mL d'eau, avant ajout d'une pointe de spatule de nickel de Raney en suspension dans l'eau. Un ballon d'hydrogène a ensuite été placé sur la réaction et le mélange a été laissé sous agitation magnétique pendant 3 heures. Le nickel a ensuite été filtré. Puis le méthanol a été éliminé à l'évaporateur rotatif et l'eau restante a été lyophilisée.

**Rf** : 0,52 (IPrOH/$NH_4OH$ 5/5 v/v).

**SM : (ESI$^+$/MeOH)** m/z : 208 $[M+H]^+$.

**SM : (ESI$^-$/MeOH)** m/z : 206 $[M-H]^-$, 413 $[2M-H]^-$.

**RMN $^1$H (400,13 MHz, $D_2O$) δ ppm :** 1,57 (m, 1H, $H_{7a}$) ; 1,88 (m, 1H, $H_{7b}$) ; 2,68 (m, 1H, $H_{6a}$) ; 2,78 (m, 1H, $H_{6b}$) ; 3,25 (s, 3H, $OCH_3$) ; 3,36 (t, 1H, $J_{4-5} = J_{4-3}$ = 9,6 Hz, $H_4$) ; 3,45 (td, 1H, $J_{5-6a} = J_{5-4}$ = 9,4 Hz, $J_{5-6b}$ = 2,7 Hz, $H_5$) ; 3,57 (dd, 1H, $J_{3-4}$ = 9,4 Hz, $J_{3-2}$ = 3,5 Hz, $H_3$) ; 3,79 (dd, 1H, $J_{2-3}$ = 3,4 Hz, $J_{2-1}$= 1,7 Hz, $H_2$) ; 4,57 (s, 1H, $H_1$).

**RMN $^{13}$C (100,62 MHz, $D_2O$) δ ppm :** 32,9 (1C, $C_7$) ; 37,7 (1C, $C_6$) ; 55,1 (1C, $OCH_3$) ; 70,2 (1C, $C_2$) ; 70,5 (1C, $C_4$) ; 70,8 (1C, $C_3$) ; 71,0 (1C, $C_5$) ; 101,2 (1C , $C_1$).

**EXEMPLE 2 : PRÉPARATION DU 6-AZIDO-6-DÉOXY-α-D-MANNOPYRANOSIDE DE MÉTHYLE (Composé I-2)**

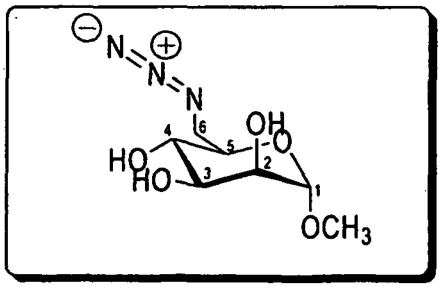

**(I-2)**

**1) première étape : Préparation du 6-azido-6-déoxy-4-O-sodiumsulfate-2,3-O-isopropylidène-α-D-mannopyranoside de méthyle (Composé 6)**

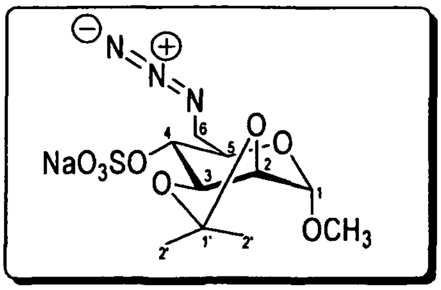

**(6)**

500 mg (1,69 mmol - 1 éq.) de 2,3-*O*-Isopropylidène-4,6-*O*-(sulfate cyclique)-α-D-mannopyranoside de méthyle (composé 3) tel qu'obtenu ci-dessus à l'issue de l'étape 1) de l'exemple 1 ont été dissous dans 3 mL de THF. 143 mg (2,20 mmol - 1,3 éq.) d'azidure de sodium ont été mis en suspension dans cette solution, puis 1,1 mL de HMPT (6,08 mmol - 3,6 éq.) ont été ajoutés. Le mélange a été laissé sous agitation magnétique à température ambiante pendant 12 heures. Le mélange réactionnel a alors été dilué avec 20 mL de $CH_2Cl_2$. Le produit a été extrait par 2 x 10 mL d'eau

distillée. Cette phase aqueuse a ensuite été lavée au $CH_2Cl_2$ jusqu'à ce que tout le HMPT soit éliminé. Après lyophilisation, le solide un peu jaune ainsi obtenu était assez pur pour être remis en réaction directement. Cependant, il peut être purifié par chromatographie sur gel de silice avec un gradient d'élution ($CH_2Cl_2$/MeOH 95/5 v/v jusqu'à $CH_2Cl_2$/MeOH 92/8 v/v) pour donner une mousse blanche.

**Rendement** : Quantitatif.

**Rf :** 0,52 ($CH_2Cl_2$/MeOH 85/15 v/v).

Le produit a été révélé de couleur orange/rouge à l'anisaldéhyde.

**SM : (ESI$^+$/MeOH)** m/z : 384 $[M+Na]^+$.

**SM : (ESI$^-$/MeOH)** m/z : 338 $[M-Na]^-$.

**RMN $^1$H (400,13 MHz, Acétone-d$_6$) δ ppm :** 1,24 et 1,41 (2s, 6H, $H_{2'}$) ; 3,31 (s, 3H, $OCH_3$) ; 3,33 (dd, 1H, $J_{6a-5}$ = 8,4 Hz, $J_{6a-6b}$ = - 13,4 Hz, $H_{6a}$) ; 4,41 (dd, 1H, $J_{6b-5}$ = 2,2 Hz, $J_{6b-6a}$ = - 13,4 Hz, $H_{6b}$) ; 3,60 (m, 1H, $H_5$) ; 4,00 (dd, 1H, $J_{2-3}$ = 5,7 Hz, $J_{2-1}$ = 0,6 Hz, $H_2$) ; 4,10 (dd, 1H, $J_{3-4}$ = 10,0 Hz, $J_{3-2}$ = 6,8 Hz, $H_3$) ; 4,25 (t, 1H, $J_{4-5}$ = $J_{4-3}$ = 6,2 Hz, $H_4$) ; 4,77 (s, 1H, $H_1$).

**RMN $^{13}$C (100,62 MHz, Acétone-d$_6$) δ ppm :** 25,8 et 27,4 (2C, $C_{2'}$) ; 52,3 (1C, $C_6$) ; 54,7 (1C, $OCH_3$) ; 69,0 (1C, $C_5$) ; 75,1 (1C, $C_3$) ; 75,8 (1C, $C_2$) ; 77,0 (1C, $C_4$) ; 98,5 (1C, $C_1$) ; 109,9 (1C, $C_{1'}$).

**2) Deuxième étape : Préparation du 6-azido-6-déoxy-α-D-mannopyranoside de méthyle (Composé I-2)**

611 mg (1,69 mmol - 1 éq.) de 6-déoxy-6-azido-4-sodiumsulfate-2,3-O-isopropylidène-α-D-manno-heptopyranoside de méthyle **(8)** obtenu ci-dessus à l'étape précédente ont été dissous dans 10 mL d'une solution constituée d'un mélange de MeOH et de THF (1/1 ; v/v). 1 g de résine Amberlyst-15 $H^+$ ont été ajoutés. Après 1 heure et 15 minutes de réaction, les résines ont été filtrées et le mélange réactionnel a été neutralisé avec une solution de $NaHCO_3$ à 5% jusqu'à pH = 8. Les solvants organiques ont été éliminés à l'évaporateur rotatif et l'eau restante a été lyophilisée. Le mélange a été repris au méthanol, et le $NaHCO_3$ insoluble a été filtré. Le produit a ensuite été purifié par chromatographie sur gel de silice avec un gradient d'élution ($CH_2Cl_2$ jusqu'à $CH_2Cl_2$/MeOH 94/6 v/v) pour donner une mousse blanche.

**Rendement** : 79 %.

**Rf** : 0,50 ($CH_2Cl_2$/MeOH 9/1 v/v).

**SM : (ESI$^+$/MeOH) m/z** : 242 $[M+Na]^+$.

**SM : (ESI$^-$/MeOH) m/z** : 218 $[M-H]^-$, 437 $[2M-H]^-$.

**RMN $^1$H (400,13 MHz, $D_2O$) δ ppm :** 3,40 (s, 3H, $OCH_3$) ; 3,54 (dd, 1H, $J_{6a-5}$ = 6,2 Hz, $J_{6a-6b}$ = - 13,3 Hz, $H_{6a}$) ; 3,60-3,73 (m, 4H, $H_{6b}$, $H_5$, $H_4$ et $H_3$) ; 3,91 (dd, 1H, $J_{2-3}$ = 3,3 Hz, $J_{2-1}$ = 1,7 Hz, $H_2$) ; 4,73 (d, 1H, $J_{1-2}$ = 1,6 Hz, $H_1$).

**RMN $^{13}$C (100,62 MHz, $D_2O$) δ ppm :** 51,4 (1C, $C_6$) ; 55,2 (1C, $OCH_3$) ; 67,8 (1C, $C_5$) ; 70,2 (1C, $C_2$) ; 70,7 (1C, $C_3$) ; 71,6 (1C, $C_4$) ; 101,4 (1C, $C_1$).

**EXEMPLE 3 : PRÉPARATION DU 6-AMINO-6-DEOXY-α-D-MANNOPYRANOSIDE DE MÉTHYLE (Composé I-3)**

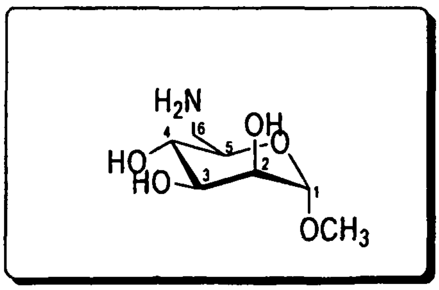

**(I-3)**

Ce composé a été préparé en utilisant une réaction de Staudinger.

130 mg (0,59 mmol - 1 éq.) de 6-déoxy-6-azido-α-D-mannopyranoside de méthyle (Composé **I-2** tel qu'obtenu ci-dessus à l'exemple 2) ont été dissous dans 3 mL de THF. 171 mg (0,65 mmol - 1,1 éq.) de triphénylphosphine et 16 μL (0,86 mmol - 1,5 éq.) d'eau ont successivement été ajoutés au mélange. Après 12 heures, le milieu réactionnel a

été concentré et déposé directement sur la colonne. La chromatographie sur gel de silice a été effectuée avec un gradient d'élution ($CH_2Cl_2$/MeOH 8/2 v/v jusqu'à $CH_2Cl_2$/MeOH 2/8 v/v) pour donner une mousse blanche.

**Rendement** : 64 %.

**Rf** : 0,10 ($CH_2Cl_2$/MeOH 8/2 v/v).

**SM : (ESI$^+$/MeOH)** m/z : 194 $[M+H]^+$, 387 $[2M+H]^+$, 409 $[2M+Na]^+$.

**SM : (ESI$^-$/MeOH)** m/z : 192 $[M-H]^-$, 385 $[2M-H]^-$.

**RMN $^1$H (400,13 MHz, $D_2O$) $\delta$ ppm :** 2,74 (dd, 1H, $J_{6a-5}$ = 7,3 Hz, $J_{6a-6b}$ = - 13,6 Hz, $H_{6a}$) ; 2,96 (dd, 1H, $J_{6b-5}$ = 2,3 Hz, $J_{6b-6a}$ = - 13,6 Hz, $H_{6b}$) ; 3,36 (s, 3H, $OCH_3$) ; 3,47 (td, 1H, $J_{5-6a}$ = $J_{5-4}$ = 8,7 Hz, $J_{5-6b}$ = 2,3 Hz, $H_5$) ; 3,51 (t, 1H, $J_{4-5}$ = $J_{4-3}$ = 9,4 Hz, $H_4$) ; 3,69 (dd, 1H, $J_{3-4}$ = 9,1 Hz, $J_{3-2}$ = 3,5 Hz, $H_3$) ; 3,88 (dd, 1H, $J_{2-3}$ = 3,5 Hz, $J_{2-1}$ = 1,7 Hz, $H_2$) ; 4,70 (d, 1H, $J_{1-2}$ = 1,6 Hz, $H_1$).

**RMN $^{13}$C (100,62 MHz, $D_2O$) $\delta$ ppm :** 41,9 (1C, $C_6$) ; 55,0 (1C, $OCH_3$) ; 68,6 (1C, $C_4$) ; 70,3 (1C, $C_2$) ; 70,8 (1C, $C_3$) ; 73,2 (1C, $C_5$) ; 101,2 (1C, $C_1$).

## EXEMPLE 4 : MISE EN ÉVIDENCE DE L'ACTIVITÉ PROANGIOGÉNIQUE DE TROIS DÉRIVÉS DE D-MANNOPYRANOSIDE DE FORMULE (I) - COMPARATIF AVEC QUATRE DÉRIVÉS DE D-MANNOPYRANOSIDE NE FAISANT PAS PARTIE DE L'INVENTION

Dans cet exemple on a étudié l'activité des composés de formule **(I-1), (I-2)** tels que préparés respectivement aux exemples 1 et 2 ci-dessus ainsi que celle du 7-phosphonato-6,7-didésoxy-D-manno-heptopyranoside de méthyle (composé **I-4)** sur l'activation de l'angiogénèse, comparativement à quatre dérivés de D-mannopyranoside (DM) ayant une activité inhibitrice de l'angiogenèse et ne faisant donc pas partie de l'invention :

**DM1 :** le (disodium)-6-phosphate-D-manno-pyranoside de méthyle ;

**DM2** : le 6,7-didéoxy-7-sulfonato-D-manno-heptopyranoside de méthyle ;

**DM3** : le 6-deoxy-6-malonate-D-mannopyranoside de méthyle ;

**DM4** : l'acide (6,7-didésoxy-D-manno-heptopyranoside de méthyle) uronique.

Cette étude a été réalisée sur des embryons de poulet selon la méthode décrite par Ribatti D. et al., Nat. Protoc., 2006, 1(1), 85-91 avec quelques modifications mineures.

### 1) Matériel et Méthode

Cette étude a été réalisée sur la membrane chorioallantoïdienne (CAM) d'embryon de poulet. La CAM est une membrane extra-embryonnaire formée le 4$^{ème}$ jour de l'incubation par la fusion du chorion et de l'allantoïde. Elle permet d'assurer les échanges gazeux entre l'embryon de poulet et l'environnement extra embryonnaire jusqu'à la naissance. Cette CAM est composée d'un réseau capillaire très épais qui forme une surface continue en contact direct avec la coquille. La prolifération capillaire rapide de cette membrane continue jusqu'au 11$^{éme}$ jour ; l'index mitotique diminue alors rapidement et le système vasculaire atteint son organisation finale au 18$^{ème}$ jour, juste avant la naissance (éclosion le 21$^{ème}$ jour).

Des oeufs fertilisés de poulet de race Leghorn blanche ont été placés dans un incubateur dès le début de l'embryogenèse où ils ont été conservés sous humidité constante à une température de 38°C. Au deuxième jour de l'incubation, une fenêtre a été ouverte dans la coquille après élimination de 2 à 3 ml d'albumine afin de détacher la CAM de la coquille. La fenêtre a ensuite été scellée avec du ruban adhésif et l'oeuf a été remis dans l'incubateur pour poursuivre son développement jusqu'au jour de l'expérience. Au 7$^{ème}$ jour, des morceaux de polymères synthétiques inertes (disques filtres de nitrocellulose de 0,4 cm de diamètre) ont été imbibés par 20 $\mu$l de chacune des solutions des composés à tester (6 mg/ml dans du PBS) puis positionnés sur la CAM. L'impact des substances testées sur l'angiogenèse a alors été observé au 12$^{ème}$ jour et l'évaluation quantitative de la réponse pro- ou anti-angiogénique a été estimée visuellement.

### 2) Résultats

Les résultats obtenus ont été photographiés et sont donnés sur la figure 1 annexée sur laquelle on peut observer que les composés **(I-1) (I-2)** et **(I-4)** ont un effet activateur sur la vascularisation des embryons de poulet. A l'inverse, les dérivés DM1, DM2 ; DM3 et DM4 ne faisant pas partie de l'invention ont un effet inhibiteur sur la vascularisation des embryons de poulets. Ces résultats démontrent que malgré une structure chimique très proche des dérivés de D-mannopyranose peuvent avoir des comportements totalement opposés sur la modulation de l'angiogenèse.

L'ensemble de ces résultats démontre clairement que les composés de formule (I) conformes à l'invention ont une action pro-angiogénique.

## Revendications

**1.** Utilisation, à titre de principe actif, d'au moins un composé de formule (I) ci-après :

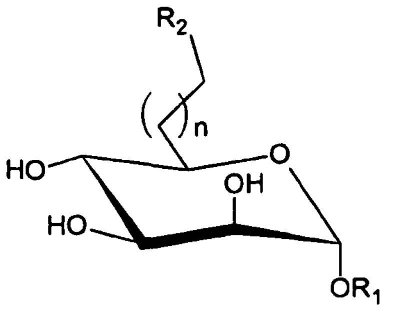

(I)

dans laquelle :

- $R_1$ représente un radical alkyle linéaire ou ramifié en $C_1$-$C_4$ ; un radical alkyle comportant un ou plusieurs groupements fonctionnels choisis parmi les groupements hydroxyle, amine, thiol, carboxyle, azide et nitrile ; un cycle hydrocarboné, saturé ou insaturé, en $C_3$-$C_6$ ; un cycle hydrocarboné, saturé ou insaturé, en $C_3$-$C_6$ comportant un ou plusieurs groupements fonctionnels choisis parmi les groupements hydroxyle, amine, alkyle en $C_1$-$C_4$, thiol, carboxyle, azide et nitrile ; un hétérocycle saturé ou insaturé comportant au moins un hétéroatome choisi parmi les atomes d'oxygène, d'azote et de souffre ;
- n est un nombre entier égal à 0 ou 1,
- $R_2$ est choisi parmi les groupements ($G_1$) à ($G_4$) suivants :

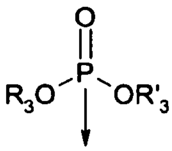

($G_1$)

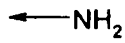

($G_2$)

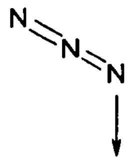

($G_3$)

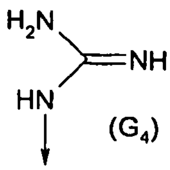

($G_4$)

dans lesquels :

* $R_3$ et $R'_3$, identiques ou différents, représentent un atome d'hydrogène ou de sodium ;
* la flèche représente le point d'attachement du groupement sur l'atome de carbone porteur de $R_2$,

pour la préparation d'une composition pharmaceutique destinée à la prévention et/ou au traitement des pathologies dépendantes d'une activation de l'angiogenèse.

**2.** Utilisation selon la revendication 1, **caractérisée par le fait que** $R_1$ représente un radical méthyle.

**3.** Utilisation selon la revendication 1, **caractérisée par le fait que** les radicaux alkyle fonctionnalisés cités pour $R_1$ sont choisis parmi les radicaux mono et dihydroxyalkyle en $C_1$-$C_4$, mono et diaminoalkyle en $C_1$-$C_4$, mono et dithioalkyle en $C_1$-$C_4$ et mono et dicarboxyalkyle en $C_1$-$C_4$.

**4.** Utilisation selon la revendication 1, **caractérisée par le fait que** les cycles hydrocarbonés cités pour $R_1$ sont choisis

parmi les cycles cyclopropane, cyclobutane, cyclopentane, cyclohexane, phényle, benzyle.

5. Utilisation selon la revendication 1, **caractérisée par le fait que** les hétérocycles cités pour $R_1$ sont choisis parmi les cycles oxadiazole, triazole, oxazole, isoxazole, imidazole, thiadiazole, pyrrole, tetrazole, furane, thiophène, pyrazole, pyrazoline, pyrazolidine, thiazole, isothiazole, pyridine, pyrimidine, pipéridine, pyranne, pyrazine et pyridazine.

6. Utilisation selon l'une quelconque des revendications précédentes, **caractérisée par le fait que** dans les composés de formule (I), lorsque n = 0, $R_2$ est un groupement $G_3$ et lorsque n = 1, $R_2$ est choisi parmi les groupements $G_1$, $G_2$ et $G_3$.

7. Utilisation selon l'une quelconque des revendications précédentes, **caractérisée par le fait que** les composés de formule (I) sont choisis parmi ceux dans lesquels $R_2$ représente un groupement $G_1$ tel que défini ci-dessus à la revendication 1, dans lequel $R_3$ et $R'_3$ sont identiques et représentent un atome de sodium.

8. Utilisation selon l'une quelconque des revendications précédentes, **caractérisée par le fait que** les composés de formule (I) sont choisis parmi :

   - le 7-amino-6,7-didésoxy-D-manno-heptopyranoside de méthyle ;
   - le 6-azido-6-déoxy-D-mannopyranoside de méthyle ;
   - le 6-amino-6-deoxy-D-mannopyranoside de méthyle ;
   - le 7-(disodium)phosphonato-6,7-didésoxy-D-manno-heptopyranoside de méthyle ;
   - le 7-phosphonato-6,7-didésoxy-D-manno-heptopyranoside de méthyle.

9. Utilisation selon la revendication 8, **caractérisée par le fait que** les composés de formule (I) sont choisis parmi le 7-(disodium)phosphonato-6,7-didésoxy-D-manno-heptopyranoside de méthyle, le 6-azido-6-déoxy-D-mannopyranoside de méthyle et le 7-amino-6,7-didésoxy-D-manno-heptopyranoside de méthyle.

10. Utilisation selon l'une quelconque des revendications précédentes, **caractérisée par le fait que** la composition pharmaceutique est destinée à la prévention et/ou au traitement des pathologies cardiovasculaires ou de l'atrophie musculaire.

11. Utilisation selon la revendication 10, **caractérisée par le fait que** la composition pharmaceutique est destinée à la prévention et/ou traitement des pathologies cardiovasculaires et qu'elle renferme en outre un ou plusieurs principes actifs additionnels choisis parmi les anticoagulants fluidifiants, les inhibiteurs du système rénine angiotensine, les béta bloquants et les inhibiteurs de l'hydroxy-méthylglutaryl-coenzyme A (HMG CoA) synthase.

12. Utilisation selon la revendication 10, **caractérisée par le fait que** la composition pharmaceutique est destinée à la prévention et/ou traitement de l'atrophie musculaire et qu'elle renferme en outre un ou plusieurs principes actifs additionnels choisis parmi la somatotrophine, l'érythropoïétine, les facteurs de croissance analogues à l'insuline et les stéroïdes.

13. Composés de formule (I'), suivante :

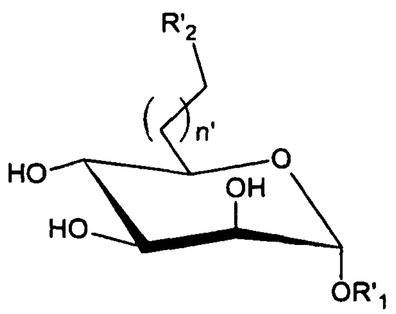

(I')

dans laquelle :

- $R'_1$ représente un radical alkyle fonctionnalisé choisi parmi les radicaux mono et dihydroxyalkyle en $C_1$-$C_4$, mono et diaminoalkyle en $C_1$-$C_4$, mono et dithioalkyle en $C_1$-$C_4$ et mono et dicarboxyalkyle en $C_1$-$C_4$ ; un cycle hydrocarboné choisi parmi les cycles cyclopropane, cyclobutane, cyclopentane, cyclohexane, phényle ; ou un hétérocycle choisi parmi les cycles oxadiazole, triazole, oxazole, isoxazole, imidazole, thiadiazole, pyrrole, tetrazole, furane, thiophène, pyrazole, pyrazoline, pyrazolidine, thiazole, isothiazole, pyridine, pyrimidine, pipéridine, pyranne, pyrazine et pyridazine ;
- n' est un nombre entier égal à 0 ou 1,
- $R'_2$ est choisi parmi les groupements ($G'_2$), ($G'_3$) et ($G'_4$) suivants :

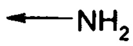

($G'_2$)

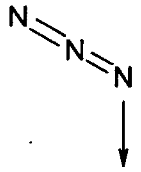

($G'_3$)

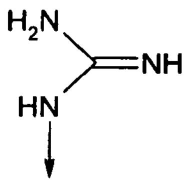

($G'_4$)

dans lesquels :

* la flèche représente le point d'attachement du groupement sur l'atome de carbone porteur de $R'_2$,

pour une utilisation à titre de médicament.

**14.** Composés selon la revendication 13, pour une utilisation à titre de médicament pour la prévention et/ou le traitement des maladies dépendant d'une activation de l'angiogénèse.

**15.** Composés selon la revendication 13, de formule (I') suivante :

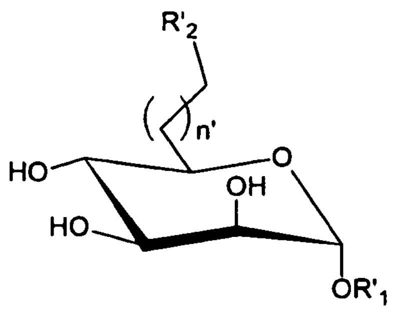

(I')

dans laquelle :

- $R'_1$ représente un radical alkyle fonctionnalisé choisi parmi les radicaux mono et dihydroxyalkyle en $C_1$-$C_4$, mono et diaminoalkyle en $C_1$-$C_4$, mono et dithioalkyle en $C_1$-$C_4$ et mono et dicarboxyalkyle en $C_1$-$C_4$ ; un cycle hydrocarboné choisi parmi les cycles cyclopropane, cyclobutane, cyclopentane, cyclohexane, phényle ; ou un hétérocycle choisi parmi les cycles oxadiazole, triazole, oxazole, isoxazole, imidazole, thiadiazole, pyrrole, tetrazole, furane, thiophène, pyrazole, pyrazoline, pyrazolidine, thiazole, isothiazole, pyridine, pyrimidine, pipéridine, pyranne, pyrazine et pyridazine ;
- n' est un nombre entier égal à 0 ou 1,
- $R'_2$ est choisi parmi les groupements ($G'_2$), ($G'_3$) et ($G'_4$) suivants :

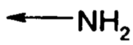

(G'$_2$)

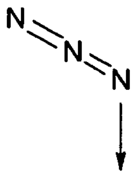

(G'$_3$)

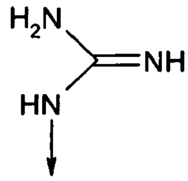

(G'$_4$)

dans lesquels :

* la flèche représente le point d'attachement du groupement sur l'atome de carbone porteur de R'$_2$,

pour une utilisation à titre de médicament pour la prévention et/ou le traitement des maladies cardiovasculaires et/ou de l'atrophie musculaire.

**16.** Composés selon l'une quelconque des revendications 13 à 15 **caractérisés par le fait que**, dans la formule (I'), R'$_1$ représente un radical alkyle fonctionnalisé choisi parmi les radicaux mono et dihydroxyalkyle en $C_1$-$C_4$, mono et diaminoalkyle en $C_1$-$C_4$, mono et dithioalkyle en $C_1$-$C_4$ et mono et dicarboxyalkyle en $C_1$-$C_4$.

**17.** Composés selon l'une quelconque des revendications 13 à 15, **caractérisés par le fait que**, dans la formule (I'), R'$_1$ représente un cycle hydrocarboné choisi parmi les cycles cyclopropane, cyclobutane, cyclopentane, cyclohexane, phényle.

**18.** Composés selon l'une quelconque des revendications 13 à 15, **caractérisés par le fait que**, dans la formule (I'), R'$_1$ représente un hétérocycle choisi parmi les cycles oxadiazole, triazole, oxazole, isoxazole, imidazole, thiadiazole, pyrrole, tetrazole, furane, thiophène, pyrazole, pyrazoline, pyrazolidine, thiazole, isothiazole, pyridine, pyrimidine, pipéridine, pyranne, pyrazine et pyridazine.

**19.** Composés selon l'une quelconque des revendications 13 à 18, **caractérisés par le fait que**, dans la formule (I'), lorsque n' = 0, R'$_2$ est un groupement G'$_3$ et lorsque n' = 1, R'$_2$ est un groupement G'$_2$ ou G'$_4$.

**20.** Composés selon l'une quelconque des revendications 13 à 19, **caractérisés par le fait que** les composés de formule (I') sont choisis parmi ceux dans lesquels R'$_2$ représente un groupement G'$_3$.

**21.** Composition pharmaceutique **caractérisée par le fait qu'**elle comprend, à titre de principe actif, au moins un composé selon la revendication 13 de formule (I') suivante :

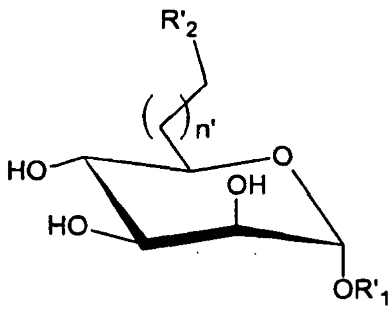

(I')

dans laquelle :

- R'$_1$ représente un radical alkyle fonctionnalisé choisi parmi les radicaux mono et dihydroxyalkyle en $C_1$-$C_4$, mono et diaminoalkyle en $C_1$-$C_4$, mono et dithioalkyle en $C_1$-$C_4$ et mono et dicarboxyalkyle en $C_1$-$C_4$ ; un cycle hydrocarboné choisi parmi les cycles cyclopropane, cyclobutane, cyclopentane, cyclohexane, phényle ; ou un hétérocycle choisi parmi les cycles oxadiazole, triazole, oxazole, isoxazole, imidazole, thiadiazole, pyrrole, tetrazole, furane, thiophène, pyrazole, pyrazoline, pyrazolidine, thiazole, isothiazole, pyridine, pyrimidine, pipéridine, pyranne, pyrazine et pyridazine ;
- n' est un nombre entier égal à 0 ou 1,
- $R'_2$ est choisi parmi les groupements ($G'_2$), ($G'_3$) et ($G'_4$) suivants :

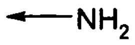

($G'_2$)

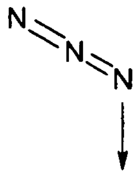

($G'_3$)

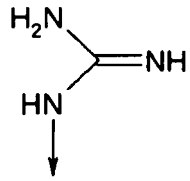

($G'_4$)

dans lesquels :

* la flèche représente le point d'attachement du groupement sur l'atome de carbone porteur de $R'_2$,

et au moins un excipient pharmaceutiquement acceptable.

**22.** Composition pharmaceutique selon la revendication 21, **caractérisée par le fait que**, dans la formule (I'), $R'_1$ représente un radical alkyle fonctionnalisé choisi parmi les radicaux mono et dihydroxyalkyle en $C_1$-$C_4$, mono et diaminoalkyle en $C_1$-$C_4$, mono et dithioalkyle en $C_1$-$C_4$ et mono et dicarboxyalkyle en $C_1$-$C_4$.

**23.** Composition pharmaceutique selon la revendication 21, **caractérisée par le fait que**, dans la formule (I'), $R'_1$ représente un cycle hydrocarboné choisi parmi les cycles cyclopropane, cyclobutane, cyclopentane, cyclohexane, phényle.

**24.** Composition pharmaceutique selon la revendication 21, **caractérisée par le fait que**, dans la formule (I'), $R'_1$ représente un hétérocycle choisi parmi les cycles oxadiazole, triazole, oxazole, isoxazole, imidazole, thiadiazole, pyrrole, tetrazole, furane, thiophène, pyrazole, pyrazoline, pyrazolidine, thiazole, isothiazole, pyridine, pyrimidine, pipéridine, pyranne, pyrazine et pyridazine.

**25.** Composition pharmaceutique selon l'une quelconque des revendications 21 à 24, **caractérisée par le fait que**, dans la formule (I'), lorsque n' = 0, $R'_2$ est un groupement $G'_3$ et lorsque n' = 1, $R'_2$ est un groupement $G'_2$ ou $G'_4$.

**26.** Composition pharmaceutique selon l'une quelconque des revendications 21 à 25, **caractérisée par le fait que** les composés de formule (I') sont choisis parmi ceux dans lesquels $R'_2$ représente un groupement $G'_3$.

**27.** Utilisation d'au moins un composé de formule (I) tel que défini à l'une quelconque des revendications 1 à 9, pour la préparation d'une composition cosmétique ou d'un complément alimentaire destiné à prévenir et/ou freiner la chute des cheveux.

**28.** Composition cosmétique **caractérisée par le fait qu'**elle comprend, dans un milieu cosmétiquement acceptable, au moins un composé de formule (I) tel que défini à l'une quelconque des revendications 1 à 9.

**29.** Complément alimentaire, **caractérisé par le fait qu'**il comprend au moins un composé de formule (I) tel que défini à l'une quelconque des revendications 1 à 9.

## Claims

**1.** Use, as an active principle, of at least one compound of the formula (I) below:

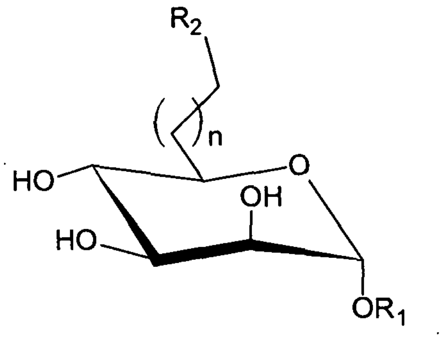

(I)

in which:

- $R_1$ represents a linear or branched $C_1$-$C_4$ alkyl radical; an alkyl radical containing one or more functional groups chosen from the hydroxyl, amine, thiol, carboxyl, azide and nitrile groups; a saturated or unsaturated $C_3$-$C_6$ hydrocarbon ring; a saturated or unsaturated $C_3$-$C_6$ hydrocarbon ring containing one or more functional groups chosen from the hydroxyl, amine, $C_1$-$C_4$ alkyl, thiol, carboxyl, azide and nitrile groups; a saturated or unsaturated heterocycle containing at least one hetero atom chosen from oxygen, nitrogen and sulfur atoms;
- n is an integer equal to 0 or 1
- $R_2$ is chosen from the following groups ($G_1$) to ($G_4$) :

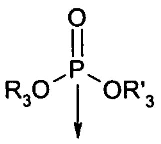

($G_1$)

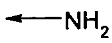

($G_2$)

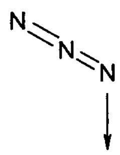

($G_3$)

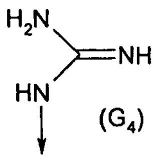

($G_4$)

in which:

* $R_3$ and $R'_3$ are identical or different and represent a hydrogen or sodium atom;
* the arrow represents the point of attachment of the group on the carbon atom carrying $R_2$,

for the preparation of a pharmaceutical composition intended for the prevention and/or treatment of pathologies dependent on an activation of angiogenesis.

**2.** Use according to claim 1, **characterized in that** $R_1$ represents a methyl radical.

**3.** Use according to claim 1, **characterized in that** the functionalized alkyl radicals mentioned for $R_1$ are chosen from the $C_1$-$C_4$ mono- and dihydroxyalkyl, $C_1$-$C_4$ mono- and diaminoalkyl, $C_1$-$C_4$ mono- and dithioalkyl and $C_1$-$C_4$ mono- and dicarboxyalkyl radicals.

**4.** Use according to claim 1, **characterized in that** the hydrocarbon rings mentioned for $R_1$ are chosen from the cyclopropane, cyclobutane, cyclopentane, cyclohexane, phenyl and benzyl rings.

**5.** Use according to claim 1, **characterized in that** the heterocycles mentioned for $R_1$ are chosen from the oxadiazole,

triazole, oxazole, isoxazole, imidazole, thiadiazole, pyrrole, tetrazole, furan, thiophene, pyrazole, pyrazoline, pyrazolidine, thiazole, isothiazole, pyridine, pyrimidine, piperidine, pyran, pyrazine and pyridazine rings.

**6.** Use according to any one of the preceding claims, **characterized in that** in the compounds of the formula (I) if n = 0 $R_2$ is a group $G_3$, and if n = 1 $R_2$ is chosen from the groups $G_1$, $G_2$ and $G_3$.

**7.** Use according to any one of the preceding claims, **characterized in that** the compounds of the formula (I) are chosen from those in which $R_2$ represents a group $G_1$ as defined above in claim 1, in which $R_3$ and $R'_3$ are identical and represent a sodium atom.

**8.** Use according to any one of the preceding claims, **characterized in that** the compounds of the formula (I) are chosen from:

- methyl 7-amino-6,7-dideoxy-D-manno-heptopyranoside;
- methyl 6-azido-6-deoxy-D-mannopyranoside;
- methyl 6-amino-6-deoxy-D-mannopyranoside;
- methyl 7-(disodium)phosphonato-6,7-dideoxy-mannoheptopyranoside;
- methyl 7-phosphonato-6,7-dideoxy-D-manno-heptopyranoside.

**9.** Use according to claim 8, **characterized in that** the compounds of the formula (I) are chosen from methyl 7-(disodium) phosphonato-6,7-dideoxy-D-manno-heptopyranoside, methyl 6-azido-6-deoxy-D-mannopyranoside and methyl 7-amino-6,7-dideoxy-D-manno-heptopyranoside.

**10.** Use according to any one of the preceding claims, **characterized in that** the pharmaceutical composition is intended for the prevention and/or treatment of cardiovascular pathologies or muscular atrophy.

**11.** Use according to claim 10, **characterized in that** the pharmaceutical composition is intended for the prevention and/or treatment of cardiovascular pathologies and **in that** it also comprises one or more additional active principles chosen from liquefying anticoagulants, inhibitors of the renin-angiotensin system, beta-blockers and inhibitors of hydroxymethylglutaryl-coenzyme A (HMG-CoA) synthase.

**12.** Use according to claim 10, **characterized in that** the pharmaceutical composition is intended for the prevention and/or treatment of muscular atrophy and **in that** it also comprises one or more additional active principles chosen from somatotropin, erythropoietin, insulin-analogous growth factors and steroids.

**13.** Compounds of the following formula (I'):

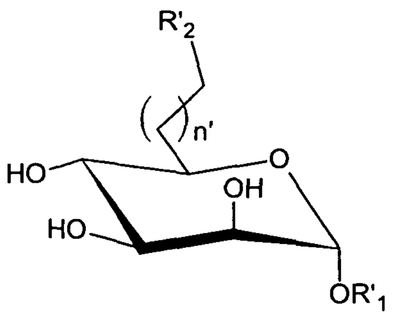

(I')

in which:

- $R'_1$ represents a functionalized alkyl radical chosen from the $C_1$-$C_4$ mono- and dihydroxyalkyl, $C_1$-$C_4$ mono- and diaminoalkyl, $C_1$-$C_4$ mono- and dithioalkyl and $C_1$-$C_4$ mono- and dicarboxyalkyl radicals; a hydrocarbon ring chosen from the cyclopropane, cyclobutane, cyclopentane, cyclohexane and phenyl rings; or a heterocycle chosen from the oxadiazole, triazole, oxazole, isoxazole, imidazole, thiadiazole, pyrrole, tetrazole, furan, thiophene, pyrazole, pyrazoline, pyrazolidine, thiazole, isothiazole, pyridine, pyrimidine, piperidine, pyran, pyrazine and pyridazine rings;
- n' is an integer equal to 0 or 1,

- $R'_2$ is chosen from the following groups ($G'_2$), ($G'_3$) and ($G'_4$) :

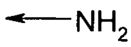 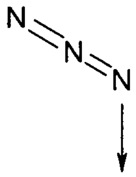 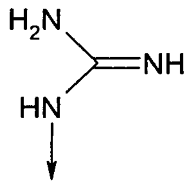

($G'_2$) ($G'_3$) ($G'_4$)

in which:

* the arrow represents the point of attachment of the group on the carbon atom carrying $R'_2$,

for a use as a medicament.

**14.** Compounds according to claim 13, for a use as a medicament for the prevention and/or the treatment of diseases dependent on an activation of angiogenesis.

**15.** Compounds according to claim 13 of the following formula (I'):

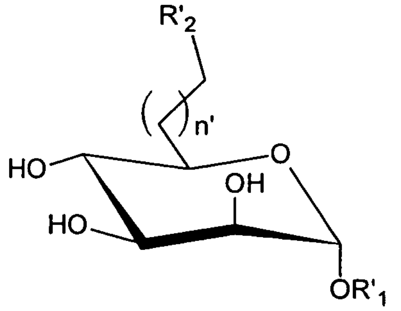

(I')

in which:

- $R'_1$ represents a functionalized alkyl radical chosen from the $C_1$-$C_4$ mono- and dihydroxyalkyl, $C_1$-$C_4$ mono- and diaminoalkyl, $C_1$-$C_4$ mono- and dithioalkyl and $C_1$-$C_4$ mono- and dicarboxyalkyl radicals; a hydrocarbon ring chosen from the cyclopropane, cyclobutane, cyclopentane, cyclohexane and phenyl rings; or a heterocycle chosen from the oxadiazole, triazole, oxazole, isoxazole, imidazole, thiadiazole, pyrrole, tetrazole, furan, thiophene, pyrazole, pyrazoline, pyrazolidine, thiazole, isothiazole, pyridine, pyrimidine, piperidine, pyran, pyrazine and pyridazine rings;
- n' is an integer equal to 0 or 1,
- $R'_2$ is chosen from the following groups ($G'_2$), ($G'_3$) and ($G'_4$):

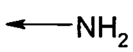 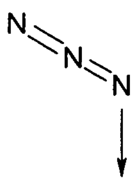 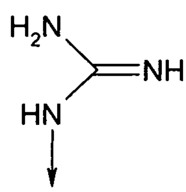

($G'_2$) ($G'_3$) ($G'_4$)

in which:

* the arrow represents the point of attachment of the group on the carbon atom carrying R’$_2$,

for a use as a medicament for the prevention and/or treatment of cardiovascular diseases and/or muscular atrophy.

**16.** Compounds according to any one of claims 13 to 15, **characterized in that** in the formula (I’) R’$_1$ represents a functionalized alkyl radical chosen from the $C_1$-$C_4$ mono- and dihydroxyalkyl, $C_1$-$C_4$ mono- and diaminoalkyl, $C_1$-$C_4$ mono- and dithioalkyl and $C_1$-$C_4$ mono- and dicarboxyalkyl radicals.

**17.** Compounds according to any one of claims 13 to 15, **characterized in that** in the formula (I’) R’$_1$ represents a hydrocarbon ring chosen from the cyclopropane, cyclobutane, cyclopentane, cyclohexane and phenyl rings.

**18.** Compounds according to any one of claims 13 to 15, **characterized in that** in the formula (I’) R’$_1$ represents a heterocycle chosen from the oxadiazole, triazole, oxazole, isoxazole, imidazole, thiadiazole, pyrrole, tetrazole, furan, thiophene, pyrazole, pyrazoline, pyrazolidine, thiazole, isothiazole, pyridine, pyrimidine, piperidine, pyran, pyrazine and pyridazine rings.

**19.** Compounds according to any one of claims 13 to 18, **characterized in that** in the formula (I’) if n’ = 0 R’$_2$ is a group G’$_3$, and if n’ = 1 R’$_2$ is a group G’$_2$ or G’$_4$.

**20.** Compounds according to any one of claims 13 to 19, **characterized in that** the compounds of the formula (I’) are chosen from those in which R’$_2$ represents a group G’$_3$.

**21.** Pharmaceutical composition, **characterized in that** it comprises as an active principle at least one compound according to claim 13 of the following formula (I’):

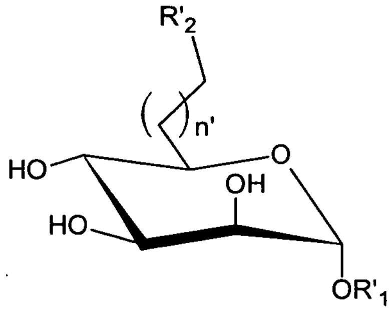

(I')

in which:

- R’$_1$ represents a functionalized alkyl radical chosen from the $C_1$-$C_4$ mono- and dihydroxyalkyl, $C_1$-$C_4$ mono- and diaminoalkyl, $C_1$-$C_4$ mono- and dithioalkyl and $C_1$-$C_4$ mono- and dicarboxyalkyl radicals; a hydrocarbon ring chosen from the cyclopropane, cyclobutane, cyclopentane, cyclohexane and phenyl rings; or a heterocycle chosen from the oxadiazole, triazole, oxazole, isoxazole, imidazole, thiadiazole, pyrrole, tetrazole, furan, thiophene, pyrazole, pyrazoline, pyrazolidine, thiazole, isothiazole, pyridine, pyrimidine, piperidine, pyran, pyrazine and pyridazine rings;
- n’ is an integer equal to 0 or 1,
- R’$_2$ is chosen from the following groups (G’$_2$), (G’$_3$) and (G’$_4$):

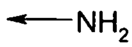

(G'$_2$)

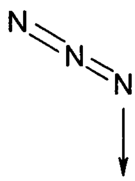

(G'$_3$)

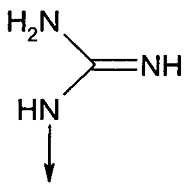

(G'$_4$)

in which:

* the arrow represents the point of attachment of the group on the carbon atom carrying $R'_2$,

and at least one pharmaceutically acceptable excipient.

**22.** Pharmaceutical composition according to claim 21, **characterized in that** in the formula (I') $R'_1$ represents a functionalized alkyl radical chosen from the $C_1$-$C_4$ mono- and dihydroxyalkyl, $C_1$-$C_4$ mono- and diaminoalkyl, $C_1$-$C_4$ mono- and dithioalkyl and $C_1$-$C_4$ mono- and dicarboxyalkyl radicals.

**23.** Pharmaceutical composition according to claim 21, **characterized in that** in the formula (I') $R'_1$ represents a hydrocarbon ring chosen from the cyclopropane, cyclobutane, cyclopentane, cyclohexane and phenyl rings.

**24.** Pharmaceutical composition according to claim 21, **characterized in that** in the formula (I') $R'_1$ represents a heterocycle chosen from the oxadiazole, triazole, oxazole, isoxazole, imidazole, thiadiazole, pyrrole, tetrazole, furan, thiophene, pyrazole, pyrazoline, pyrazolidine, thiazole, isothiazole, pyridine, pyrimidine, piperidine, pyran, pyrazine and pyridazine rings.

**25.** Pharmaceutical composition according to any one of claims 21 to 24, **characterized in that** in the formula (I') if n' = 0 $R'_2$ is a group $G'_3$, and if n' = 1 $R'_2$ is a group $G'_2$ or $G'_4$.

**26.** Pharmaceutical composition according to any one of claims 21 to 25, **characterized in that** the compounds of the formula (I') are chosen from those in which $R'_2$ represents a group $G'_3$.

**27.** Use of at least one compound of the formula (1) as defined in any one of claims 1 to 9 for the preparation of a cosmetic composition or of a food supplement intended for the prevention and/or slowing down of hair loss.

**28.** Cosmetic composition, **characterized in that** it comprises at least one compound of the formula (I) as defined in any one of claims 1 to 9 in a cosmetically acceptable medium.

**29.** Food supplement, **characterized in that** it comprises at least one compound of the formula (I) as defined in any one of claims 1 to 9.

## Patentansprüche

**1.** Verwendung wenigstens einer Verbindung der folgenden Formel (I):

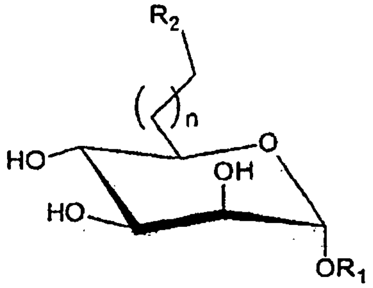

(I)

in der:

- $R_1$ einen linearen oder verzweigten $C_1$-$C_4$-Alkylrest; einen Alkylrest, der eine oder mehrere funktionelle Gruppierungen, ausgewählt aus Hydroxyl-, Amin-, Thiol-, Carboxyl-, Azid- und Nitril-Gruppierungen, umfasst; einen gesättigten oder ungesättigten $C_3$-$C_6$-Kohlenwasserstoffring; einen gesättigten oder ungesättigten $C_3$-$C_6$-Kohlenwasserstoffring, der eine oder mehrere funktionelle Gruppierungen, ausgewählt aus Hydroxyl-, Amin-, $C_1$-$C_4$-Alkyl-, Thiol-, Carboxyl-, Azid- und Nitril-Gruppierungen, umfasst; einen gesättigten oder ungesättigten Heterocyclus, der wenigstens ein Heteroatom, ausgewählt aus den Sauerstoff-, Stickstoff- und Schwefelatomen,

umfasst, darstellt;
- n eine ganze Zahl von 0 oder 1 darstellt;
- $R_2$ ausgewählt ist aus den folgenden Gruppierungen ($G_1$) bis ($G_4$) :

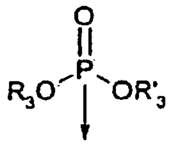
($G_1$)

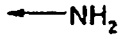
($G_2$)

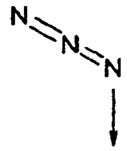
($G_3$)

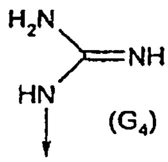
($G_4$)

in denen:

* $R_3$ und $R'_3$, die gleich oder unterschiedlich sind, ein Wasserstoff- oder Natriumatom darstellen;
* die Pfeilspitze den Anbindungspunkt der Gruppierung an das Kohlenstoffatom, das Träger von $R_2$ ist, darstellt,

als Wirkstoff für die Herstellung einer pharmazeutischen Zusammensetzung, die zur Prävention und/oder Behandlung von Pathologien bestimmt ist, die von einer Aktivierung der Angiogenese abhängig sind.

**2.** Verwendung gemäß Anspruch 1, **dadurch gekennzeichnet , dass** $R_1$ einen Methylrest darstellt.

**3.** Verwendung gemäß Anspruch 1, **dadurch gekennzeichnet, dass** die für $R_1$ genannten funktionalisierten Alkylreste aus Mono- und Dihydroxy-$C_1$-$C_4$-alkyl, Mono- und Diamino-$C_1$-$C_4$-alkyl, Mono- und Dithio-$C_1$-$C_4$-alkyl und Mono- und Dicarboxy-$C_1$-$C_4$-alkyl ausgewählt sind.

**4.** Verwendung gemäß Anspruch 1, **dadurch gekennzeichnet , dass** die für $R_1$ genannten Kohlenwasserstoffringe aus den Cyclopropan-, Cyclobutan-, Cyclopentan-, Cyclohexan-, Phenyl-, Benzylringen ausgewählt sind.

**5.** Verwendung gemäß Anspruch 1, dadurch **gekennzeich**n e t , dass die für $R_1$ genannten Heterocyclen aus den Ringen Oxadiazol, Triazol, Oxazol, Isoxazol, Imidazol, Thiadiazol, Pyrrol, Tetrazol, Furan, Thiophen, Pyrazol, Pyrazolin, Pyrazolidin, Thiazol, Isothiazol, Pyridin, Pyrimidin, Piperidin, Pyran, Pyrazin und Pyridazin ausgewählt sind.

**6.** Verwendung gemäß einem der vorangehenden Ansprüche, **dadurch gekennzeichnet, dass**, wenn in den Verbindungen der Formel (I) n = 0, $R_2$ eine Gruppierung $G_3$ ist, und wenn n = 1, $R_2$ aus den Gruppierungen $G_1$, $G_2$ und $G_3$ ausgewählt ist.

**7.** Verwendung gemäß einem der vorangehenden Ansprüche, **dadurch gekennzeichnet, dass** die Verbindungen der Formel (I) unter denen ausgewählt sind, in denen $R_2$ eine Gruppierung $G_1$, wie oben in Anspruch 1 definiert, in der $R_3$ und $R'_3$ gleich sind und ein Natriumatom darstellen, darstellt.

**8.** Verwendung gemäß einem der vorangehenden Ansprüche, **dadurch gekennzeichnet, dass** die Verbindungen der Formel (I) ausgewählt sind aus:

- Methyl-7-amino-6,7-didesoxy-D-manno-heptopyranosid;
- Methyl-6-azido-6-desoxy-D-mannopyranosid;
- Methyl-6-amino-6-desoxy-D-mannopyranosid;
- Methyl-7-(dinatrium)phosphonato-6,7-didesoxy-D-mannoheptopyranosid;
- Methyl-7-phosphonato-6,7-didesoxy-D-manno-heptopyranosid.

**9.** Verwendung gemäß Anspruch 8, **dadurch gekennzeichnet , dass** die Verbindungen der Formel (I) aus Methyl-7-(dinatrium)phosphonato-6,7-didesoxy-D-manno-heptopyranosid, Methy-6-azido-6-desoxy-D-mannopyranosid und Methyl-7-amino-6,7-didesoxy-D-manno-heptopyranosid ausgewählt sind.

**10.** Verwendung gemäß einem der vorangehenden Ansprüche, **dadurch gekennzeichnet, dass** die pharmazeutische Zusammensetzung zur Prävention und/oder Behandlung von kardiovaskulären Pathologien und Muskelatrophie bestimmt ist.

**11.** Verwendung gemäß Anspruch 10, **dadurch gekennzeichnet, dass** die pharmazeutische Zusammensetzung zur Prävention und/oder Behandlung von kardiovaskulären Pathologien bestimmt ist und dass sie außerdem einen zusätzlichen Wirkstoff oder mehrere zusätzliche Wirkstoffe, ausgewählt aus verdünnenden Antikoagulanzien, Inhibitoren des Renin-Angiotensin-Systems, Beta-Blockern und Inhibitoren der Hydroxymethylglutaryl-Coenzym-A (HMG-CoA)-Synthase, umfasst.

**12.** Verwendung gemäß Anspruch 10, **dadurch gekennzeichnet, dass** die pharmazeutische Zusammensetzung zur Prävention und/oder Behandlung von Muskelatrophie bestimmt ist und dass sie außerdem einen weiteren Wirkstoff oder mehrere weitere Wirkstoffe, ausgewählt aus Somatotrophin, Erythropoietin, Insulin-ähnlichen Wachstumsfaktoren und Steroiden, umfasst.

**13.** Verbindungen der folgenden Formel (I'):

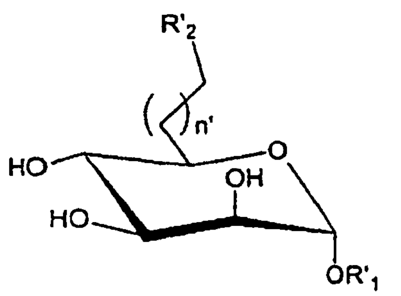

(I')

in der:

- $R'_1$ einen funktionalisierten Alkylrest, ausgewählt aus den Resten Mono- und Dihydroxy-$C_1$-$C_4$-alkyl, Mono- und Diamino-$C_1$-$C_4$-alkyl, Mono- und Dithio-$C_1$-$C_4$-alkyl und Mono- und Dicarboxy-$C_1$-$C_4$-alkyl; einen Kohlenwasserstoffring, ausgewählt aus den Ringen Cyclopropan, Cyclobutan, Cyclopentan, Cyclohexan, Phenyl, oder einen Heterocyclus, ausgewählt aus den Ringen Oxadiazol, Triazol, Oxazol, Isoxazol, Imidazol, Thiadiazol, Pyrrol, Tetrazol, Furan, Thiophen, Pyrazol, Pyrazolin, Pyrazolidin, Thiazol, Isothiazol, Pyridin, Pyrimidin, Piperidin, Pyran, Pyrazin und Pyridazin, darstellt;
- n' eine ganze Zahl von 0 bis 1 ist;
- $R'_2$ aus den folgenden Gruppierungen ($G'_2$), ($G'_3$) und ($G'_4$) ausgewählt ist:

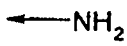

($G'_2$)

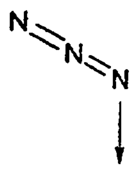

($G'_3$)

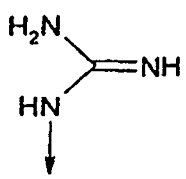

($G'_4$)

in denen:

* die Pfeilspitze den Anbindungspunkt der Gruppierung an das Kohlenstoffatom, das Träger von $R'_2$ ist, darstellt,

zur Verwendung als Medikament.

**14.** Verbindungen gemäß Anspruch 13 für eine Verwendung als Medikament zur Prävention und/oder Behandlung von Krankheiten, die von einer Aktivierung der Angiogenese abhängen.

**15.** Verbindungen gemäß Anspruch 13 der folgenden Formel (I'):

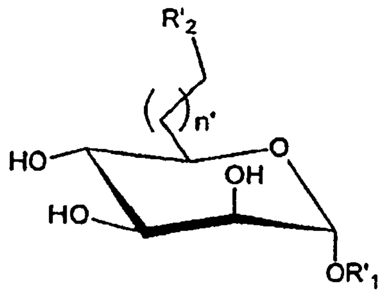

(I')

in der:

- $R'_1$ einen funktionalisierten Alkylrest, ausgewählt aus den Resten Mono- und Dihydroxy-$C_1$-$C_4$-alkyl, Mono- und Diamino-$C_1$-$C_4$-alkyl, Mono- und Dithio-$C_1$-$C_4$-alkyl und Mono- und Dicarboxy-$C_1$-$C_4$-alkyl; einen Kohlenwasserstoffring, ausgewählt aus den Ringen Cyclopropan, Cyclobutan, Cyclopentan, Cyclohexan, Phenyl, und einen Heterocyclus, ausgewählt aus den Ringen Oxadiazol, Triazol, Oxazol, Isoxazol, Imidazol, Thiadiazol, Pyrrol, Tetrazol, Furan, Thiophen, Pyrazol, Pyrazolin, Pyrazolidin, Thiazol, Isothiazol, Pyridin, Pyrimidin, Piperidin, Pyran, Pyrazin und Pyridazin, darstellt,
- n' eine ganze Zahl von 0 oder 1 ist;
- $R'_2$ ausgewählt ist aus den folgenden Gruppierungen ($G'_2$), ($G'_3$) und ($G'_4$) :

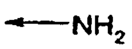

($G'_2$)

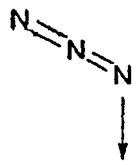

($G'_3$)

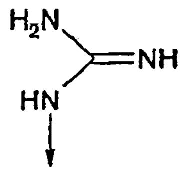

($G'_4$)

in denen:

* die Pfeilspitze den Anbindungspunkt der Gruppierung an das Kohlenstoffatom, das Träger von $R'_2$ ist, darstellt;

für eine Verwendung als Medikament zur Prävention und/oder Behandlung von kardiovaskulären Krankheiten und/oder Muskelatrophie.

**16.** Verbindungen gemäß einem der Ansprüche 13 bis 15, **dadurch gekennzeichnet, dass** in der Formel (I') $R'_1$ einen funktionalisierten Alkylrest, ausgewählt aus den Resten Mono- und Dihydroxy-$C_1$-$C_4$-alkyl, Mono- und Diamino-$C_1$-$C_4$-alkyl, Mono- und Dithio-$C_1$-$C_4$-alkyl und Mono- und Dicarboxy-$C_1$-$C_4$-alkyl, darstellt.

**17.** Verbindungen gemäß einem der Ansprüche 13 bis 15, **dadurch gekennzeichnet,** in der Formel (I') $R'_1$ einen Kohlenwasserstoffring darstellt, der aus den Ringen Cyclopropan, Cyclobutan, Cylcopentan, Cyclohexan, Phenyl ausgewählt ist.

**18.** Verbindungen gemäß einem der Ansprüche 13 bis 15, **dadurch gekennzeichnet, dass** in der Formel (I') $R'_1$ einen Heterocyclus darstellt, der aus den Ringen Oxadiazol, Triazol, Oxazol, Isoxazol, Imidazol, Thiadiazol, Pyrrol, Tetrazol, Furan, Thiophen, Pyrazol, Pyrazolin, Pyrazolidin, Thiazol, Isothiazol, Pyridin, Pyrimidin, Piperidin, Pyran, Pyrazin und Pyridazin ausgewählt ist.

**19.** Verbindungen gemäß einem der Ansprüche 13 bis 18, **dadurch gekennzeichnet, dass**, wenn in der Formel (I') n' = 0, $R'_2$ eine Gruppierung $G'_3$ ist, und wenn n' = 1, $R'_2$ eine Gruppierung $G'_2$ oder $G'_4$ ist.

**20.** Verbindungen gemäß einem der Ansprüche 13 bis 19, **dadurch gekennzeichnet, dass** die Verbindungen der Formel (I') aus denen ausgewählt sind, in denen $R'_2$ eine Gruppierung $G'_3$ darstellt.

**21.** Pharmazeutische Zusammensetzung, **dadurch gekennzeichnet, dass** sie als Wirkstoff wenigstens eine Verbindung gemäß Anspruch 13 der folgenden Formel (I'):

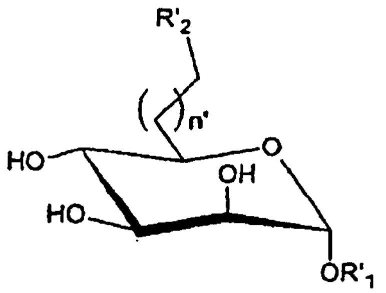

(I')

in der:

- $R'_1$ einen funktionalisierten Alkylrest, ausgewählt aus den Resten Mono- und Dihydroxy-$C_1$-$C_4$-alkyl, Mono- und Diamino-$C_1$-$C_4$-alkyl, Mono- und Dithio-$C_1$-$C_4$-alkyl und Mono- und Dicarboxy-$C_1$-$C_4$-alkyl; einen Kohlenwasserstoffring, ausgewählt aus den Ringen Cyclopropan, Cyclobutan, Cyclopentan, Cyclohexan, Phenyl, oder einen Heterocyclus, ausgewählt aus den Ringen Oxadiazol, Triazol, Oxazol, Isoxazol, Imidazol, Thiadiazol, Pyrrol, Tetrazol, Furan, Thiophen, Pyrazol, Pyrazolin, Pyrazolidin, Thiazol, Isothiazol, Pyridin, Pyrimidin, Piperidin, Pyran, Pyrazin und Pyridazin, darstellt;
- n' eine ganze Zahl von 0 bis 1 ist;
- $R'_2$ aus den folgenden Gruppierungen ($G'_2$), ($G'_3$) und ($G'_4$) ausgewählt ist:

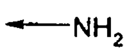

($G'_2$)

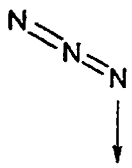

($G'_3$)

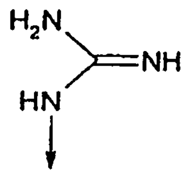

($G'_4$)

in denen:

* die Pfeilspitze den Anbindungspunkt der Gruppierung an das Kohlenstoffatom, das Träger von $R'_2$ ist, darstellt,

und wenigstens ein pharmazeutisch verträgliches Exzipiens umfasst.

**22.** Pharmazeutische Zusammensetzung gemäß Anspruch 21, **dadurch gekennzeichnet, dass** in der Formel (I') $R'_1$ einen funktionalisierten Alkylrest, ausgewählt aus den Resten Mono- und Dihydroxy-$C_1$-$C_4$-alkyl, Mono- und Diamino-$C_1$-$C_4$-alkyl, Mono- und Dithio-$C_1$-$C_4$-alkyl und Mono- und Dicarboxy-$C_1$-$C_4$-alkyl, darstellt.

**23.** Pharmazeutische Zusammensetzung gemäß Anspruch 21, **dadurch gekennzeichnet, dass** in der Formel (I') $R'_1$ einen Kohlenwasserstoffring darstellt, der aus den Ringen Cyclopropan, Cyclobutan, Cylcopentan, Cyclohexan,

Phenyl ausgewählt ist.

**24.** Pharmazeutische Zusammensetzung gemäß Anspruch 21, **dadurch gekennzeichnet, dass** in der Formel (I') $R'_1$ einen Heterocyclus darstellt, der aus den Ringen Oxadiazol, Triazol, Oxazol, Isoxazol, Imidazol, Thiadiazol, Pyrrol, Tetrazol, Furan, Thiophen, Pyrazol, Pyrazolin, Pyrazolidin, Thiazol, Isothiazol, Pyridin, Pyrimidin, Piperidin, Pyran, Pyrazin und Pyridazin ausgewählt ist.

**25.** Pharmazeutische Zusammensetzung gemäß einem der Ansprüche 21 bis 24, **dadurch gekennzeichnet, dass**, wenn in der Formel (I') n' = 0, $R'_2$ eine Gruppierung $G'_3$ ist, und wenn n' = 1, $R'_2$ eine Gruppierung $G'_2$ oder $G'_4$ ist.

**26.** Pharmazeutische Zusammensetzung gemäß einem der Ansprüche 21 bis 25, **dadurch gekennzeichnet, dass** die Verbindungen der Formel (I') aus denen ausgewählt sind, in denen $R'_2$ eine Gruppierung $G'_3$ darstellt.

**27.** Verwendung wenigstens einer Verbindung der Formel (I), wie sie in einem der Ansprüche 1 bis 9 definiert ist, für die Herstellung einer kosmetischen Zusammensetzung oder eines Nahrungsergänzungsmittels, das dazu bestimmt ist, Haarausfall zu verhindern und/oder zu verringern.

**28.** Kosmetische Zusammensetzung, **dadurch gekennzeichnet , dass** sie in einem kosmetisch verträglichen Medium wenigstens eine Verbindung der Formel (I), wie sie in einem der Ansprüche 1 bis 9 definiert ist, umfasst.

**29.** Nahrungsergänzungsmittel, **dadurch gekennzeichnet , dass** es wenigstens eine Verbindung der Formel (I), wie sie in einem der Ansprüche 1 bis 9 definiert ist, umfasst.

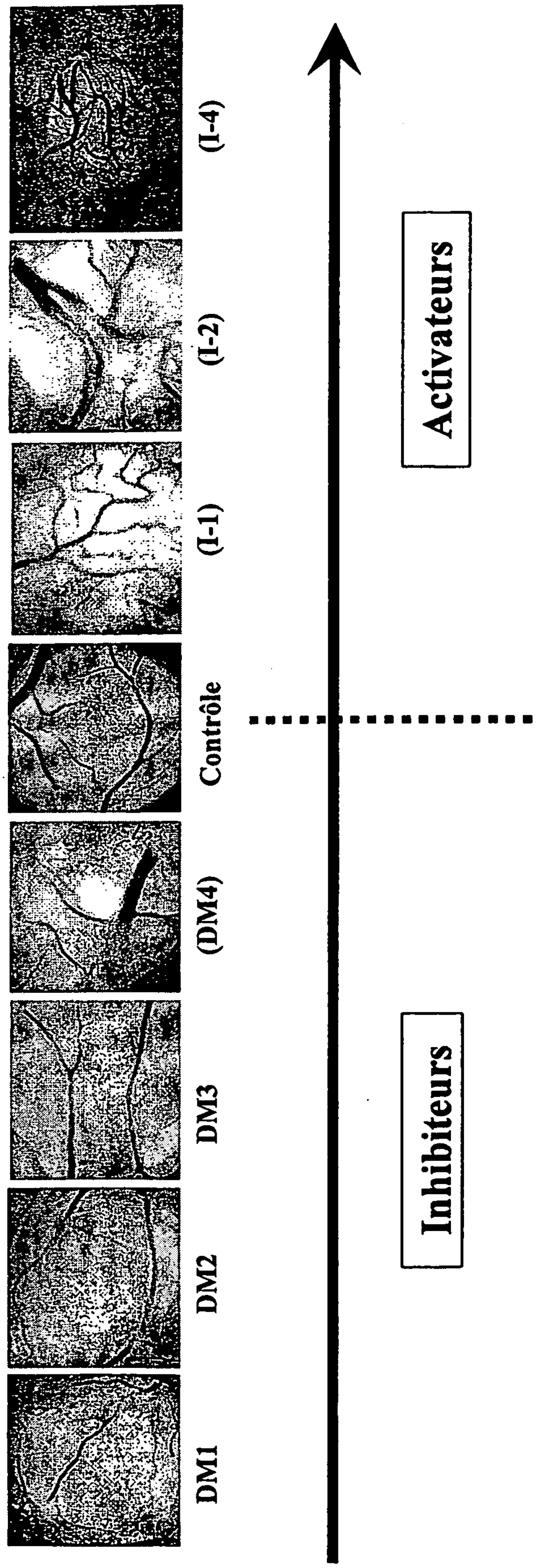


FIGURE 1

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- WO 9958126 A


### Littérature non-brevet citée dans la description


- **LAZAROUS DF ; SHOU M ; STIBER JA et al.** *Cardiovasc Res,* 1997, vol. 36, 78-85
- **CLAVEL, C. et al.** *Il Farmaco,* 2005, vol. 60, 721-725
- **VAN DER KLEIN P.A.M. et al.** *Carbohydr. Res.,* 1992, vol. 224, 193-200
- **KHANJIN N.A.** *Tetrahedr. Lett.,* 2002, vol. 43, 4017-4020
- **RIBATTI D. et al.** *Nat. Protoc.,* 2006, vol. 1 (1), 85-91